# EUROPÄISCHE PATENTSCHRIFT

(11) **EP 1 934 356 B1**
(45) Veröffentlichungstag und Bekanntmachung des Hinweises auf die Patenterteilung: **18.08.2010**
(21) Anmeldenummer: 06806235.5
(22) Anmeldetag: 12.10.2006
(51) Int. Cl.: C12N 15/82, C12N 15/53, A01H 5/00

(54) **ASTAXANTHINBIOSYNTHESE IN EUKARYOTEN**
ASTAXANTHINE BIOSYNTHESIS IN EUKARYOTES
BIOSYNTHESE D'ASTAXANTHINE CHEZ DES EUCARYOTES

(30) Priorität: 13.10.2005 DE 102005049072
(43) Veröffentlichungstag der Anmeldung: 25.06.2008
(73) Patentinhaber: Peter und Traudl Engelhorn-Stiftung zur Förderung der Biotechnologie und Gentechnik, 70569 Stuttgart (DE)
(72) Erfinder: STEINBRENNER, Jens, 78467 Konstanz (DE)
(74) Vertreter: Dey, Michael
(86) Internationale Anmeldenummer: PCT/EP2006/009881
(87) Internationale Veröffentlichungsnummer: WO 2007/042304

(56) Entgegenhaltungen:
- WO-A-2004/007691
- CHAMOVITZ D ET AL: "Molecular and biochemical characterization of herbicide-resistant mutants of cyanobacteria reveals that phytoene desaturation is a rate-limiting step in carotenoid biosynthesis" JOURNAL OF BIOLOGICAL CHEMISTRY, AMERICAN SOCIETY OF BIOLOCHEMICAL BIOLOGISTS, BIRMINGHAM,, US, Bd. 268, Nr. 23, 15. August 1993 (1993-08-15), Seiten 17348-17353, XP002400153 ISSN: 0021-9258 in der Anmeldung erwähnt
- CHEN YONG ET AL: "Screening and characterization of astaxanthin-hyperproducing mutants of Haematococcus pluvialis." BIOTECHNOLOGY LETTERS, Bd. 25, Nr. 7, April 2003 (2003-04), Seiten 527-529, XP002424020 ISSN: 0141-5492
- STEINBRENNER JENS ET AL: "Regulation of two carotenoid biosynthesis genes coding for phytoene synthase and carotenoid hydroxylase during stress-induced astaxanthin formation in the green alga Haematococcus pluvialis" PLANT PHYSIOLOGY (ROCKVILLE), Bd. 125, Nr. 2, Februar 2001 (2001-02), Seiten 810-817, XP002424021 ISSN: 0032-0889 in der Anmeldung erwähnt

## Beschreibung

Die Erfindung betrifft einen DNA-Vektor, der (a) eine DNA-Sequenz, die für das Protein Phytoen-Desaturase, welches an einer Position durch einen Resistenz vermittelnden Aminosäureseaustausch verändert ist, codiert, und (b) eine Multiple Cloning Site, in die eine beliebige DNA-Sequenz einkloniert werden kann, besitzt, dessen Verwendung zur Transformation von eukaryotischen Zellen und Verfahren zur Transformation und so hergestellte transgene Pflanzenzellen.

### Hintergrund der Erfindung

Carotinoide sind Pigmente, die in allen photosynthetischen Organismen vorkommen. Sie spielen als Komponenten des photosynthetischen Reaktionszentrums und beim Schutz gegen photooxydativen Schaden eine wichtige Rolle.

Der Carotinoid-Biosyntheseweg läuft von Geranyl-Geranyl-Diphosphat (GGDP) bis Astaxanthin. Durch die Kondensation von zwei Molekülen Geranyl-Gerany-Pyrophosphat bildet die Phytoen-Synthase (PSY) den C40-Körper Phytoen. Ausgehend von Phytoen synthetisiert die Phytoen-Desaturase (PDS) durch Abspaltung von Protonen und den Einbau von zwei Doppelbindungen ξ-Carotin. Das Zwischenprodukt dieses Syntheseschrittes ist Phytofluen. ξ-Carotin wird dann wieder in einer zweistufigen Desaturierungsreaktion über das Zwischenprodukt Neurosporin in Lycopin umgewandelt. Das hierfür verantwortlichen Enzym ist die ξ-Carotin-Desaturase (ZDS). Lycopin wird über eine Lycopin-Cyclase (LCYB) in β-Carotin umgewandelt. Ausgehend von β-Carotin sind an der Bildung von Astaxanthin zwei weitere Enzyme beteiligt. Einerseits fügt die β-Carotin-Ketolase (BKT) an der 4- und 4'-Position je eine Ketogruppe ein. Andererseits wird über die Carotinoid-Hydroxylase (CHY) je eine Hydroxylgruppe an 3- und 3'-Position an den lonenring des Astaxanthinvorläufers angehängt.

Durch die Überexpression einer bakteriellen Phytoen-Synthase aus *Eriwinia uredovora* konnte die Carotinoid-Biosynthese in transgenen Tomatenpflanzen beeinflußt bzw. stimuliert und die synthetisierte Carotinoidmenge damit um das 2-4 fache gesteigert werden (Fraser, Romer et al. 2002).

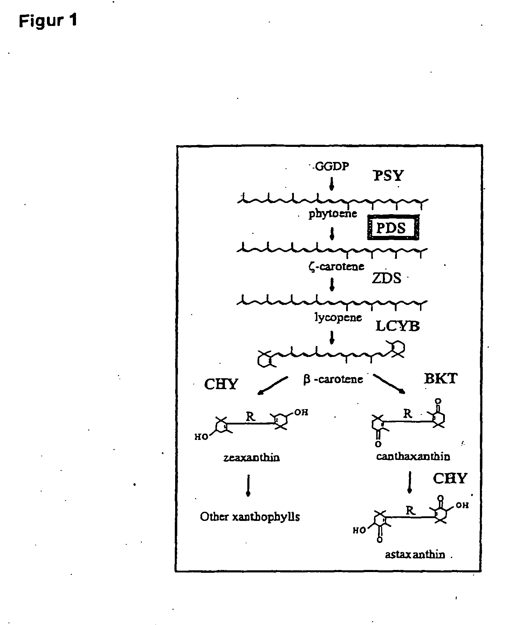

Die zentrale Schlüsselstellung im Biosyntheseweg der Carotinoide wird aber vom Enzym Phytoen-Desaturase eingenommen (Fig. 1). Die *pds*-Gene, welche für Phytoen-Desaturase codieren, wurden aus Cyanobakterien (Chamovitz et al.. 1991; Martínez-Férez and Vioque, 1992; Martinez-Férez et al., 1994) und höheren Pflanzen (Bartley et al., 1991; Pecker et al., 1992) kloniert und erfolgreich in *E. coli* überexprimiert. Als reversibler, nicht kompetitiver Inhibitor der Phytoen-Desaturase ist das Bleichherbizid Norflurazon bekannt. Für das Cyanobakterium *Synechococcus* sind mutierte Formen der Phytoen-Desaturase beschrieben worden, die dem Bakterium eine Resistenz gegenüber Norflurazon verleihen. Die Resistenzverleihenden Mutationen beruhen dabei auf jeweils einem einzigen Aminosäureaustausch. Im Rahmen der in *Synechococcus* durchgeführten Mutationsstudien wurde Resistenzen für folgende Aminosäureseaustausche gefunden: Arg195Pro; Leu320Pro; Va1403Gky; Leu437Arg (Linden et al. 1990; Chamovitz et al., 1993). Obwohl Noflurazon und andere derartige Bleichherbizide seit längerer Zeit zur Unkrautbekämpfung verwendet werden, konnten bis jetzt keine resistenten natürlich vorkommenden Pflanzen isoliert werden.

WO2004/007691 offenbart Vektoren umfassend eine DNA-Sequenz jeweils codierend für Phytoen-Desatorase, welche in einer Position eine Resistenz-' vermittelnde Aminosäuresubstitution aufweist. Die gemäß WO2004/007691 offenbarten Sequenzen stammen aus den mehrzelligen Pflanzen Hydrilla, Sojabohne, Mais oder Reis.

Von den Intermediaten und Produkten des Carotinoid-Bisyntheseweges ist insbesondere das Ketocarotinoid Astaxanthin von kommerzieller Bedeutung. Astaxanthin besitzt eine höhere Antioxidanz-Aktivität als andere Zwischenprodukte des Carotinoid-Biosynthesewegs. Astaxanthin wirkt als Quencher gegen freie Radikale und aktive Sauerstoffspezies (Kobayashi und Sakamoto, 1999), als Verstärker von Immunantworten (Jyonouchi et al., 1995) und als Antikrebsmittel (Tanaka et al., 1994,1995). Aufgrund seiner natürlichen Wirkung als potentes Antioxidanz wird Astaxanthin auch als Nahrungsmittelergänzungsstoff eingesetzt. Als Futtermitteladditiv mit Farbstoffwirkung wird es in der Fischzucht verwendet.

Einige Grünalgen wie z.B. *Dunaliella bardawil* und *Haematococcus pluvialis* besitzen die einzigartige Fähigkeit, Carotinoide unter Stress-Bedingungen zu akkumulieren. Dabei ist insbesondere *H. pluvialis* für die natürliche Herstellung von Astaxanthin geeignet. *H. pluvialis* kann Astaxanthin auf bis zu 4% ihres Trockengewicht akkumulieren. In der kommerziellen Herstellung von Astaxanthin spielt *H. pluvialis* insbesondere deshalb eine zentrale Rolle, da die chemische Syntheseroute zur Produktion von Astaxanthin zu den aufwendigsten zählt, die zur Herstellung eines Wirkstoffs kommerziell eingesetzt werden.

Die genetische Transformation von Grünalgen wurde für *Chlamydomonas reinhardtii* und einige Arten von *Chlorella* mehrfach beschrieben (Kindle 1990; Lumbreras et al. 1998; Hawkins und Nakamura, 1999; Kim et al. 2002). Die beiden Algen-Spezies gelten aufgrund ihres Stoffwechsels für die natürliche Produktion von Carotinoiden im Großmaßstab als weniger interessant.

Die genetische Transormation von *H. pluvialis* wurde durch Teng et al. (2003) beschrieben. Durch Beschuß mit Mikroteilchen wurde das β-Galactosidase Reporter-Gen la*cZ* unter Kontrolle des SV40 Promotors in das Algengenom integriert. Erfolgreich transformierte Algenzellen müssen unter zuhilfenahme optischer Mittel einzeln delektiert und selektiert werden.

Mit dem lacZ-Gen transformierte Algenzellen besitzen keine Resistenz gegenüber toxisch bzw. dominant wirkenden Selektionsmitteln

Obwohl die Astaxanthin-Biosynthese während der letzten 10 Jahre eingehend untersucht wurde (Lu et al., 1995; Fraser et al., 1998), gestaltet sich die Herstellung von Astaxanthin durch *H. pluvialis* im biotechnologischen Großmaßstab noch immer problematisch, da unter anderem die Zellteilung während der Astaxanthin-Biosynthese inhibiert wird (Boussiba und von Vonshak, 1991).

Es ist eine Aufgabe dieser Erfindung, einen DNA-Vektor bereitzustellen, der verwendet werden kann, um das Genom von Eukaryoten Zellen, insbesondere von Algen wie *H. pluvialis* in geeignter Weise genetisch zu verändern, um so die *in vivo* Synthese natürlicher Carotinoide und Isoprenoide und insbesondere die Astaxanthin-Biosynthese beeinflussen bzw. steigern zu können. Es ist eine weitere Aufgabe der Erfindung, einen Vektor bereitzustellen, der als dominant selektiver Marker für Transformation in Eukaryoten Zellen, insbesondere von Algen wie *H. pluvialis* eingesetzt werden kann und der es ermöglicht erfolgreich transformierte Eukaryoten Zellen, insbesondere von Algen wie *H. pluvialis* Zellen in einfacher Weise zu selektieren.

Diese Aufgabe löst die Erfindung durch einen Vektor umfassend umfassend (a) eine DNA-Sequenz, die für das Protein Phytoen-Desaturase, welches an einer Position eine Resistenz gegenüber Herbiziden vermittelnde Aminosäuresubstitution besitzt, codiert, und (b) eine Multiple Cloning Site, in die eine beliebige DNA-Sequenz einkloniert werden kann, wobei die das Protein Phytoen-Desaturase codierende DNA-Sequenz unter Beücksichtigung der Degeneration des genetischen Codes ein Protein gemäß SEQ ID NO:2 kodiert, wobei das Protein gemäß SEQ ID NO:2 einen Aminosäureaustausch von Leucin zu Arginin an Position 504 aufweist.

Die erfindungsgemäßen Vektoren umfassen dabei jegliche DNA-Moleküle, welche als Vehikel verwendet werden können, um mit deren Hilfe Fremd-DNA in eine Zeile einzuschleußen. Sie schließen Cosmide, Phagen, Viren, YACs, BACs, linearere DNA-Moleküle und insbesondere ringförmige Plasmide ein.

Im Rahmen der Erfindung wurde die DNA-Sequenz des Enzyms Phytoen-Desaturase (PDS) aus *H. pluvialis* aus einer genomischen DNA-Bibliothek isoliert und sequenziert (SEQ ID NO:1). Die entsprechende Proteinsequenz der Phytoen-Desaturase aus *H. pluvialis* ist in SEQ ID NO:2 gezeigt. Der Gegenstand der Erfindung umfasst alle Nukleinsäuresequenzen, die unter Berücksichtigung der Degeneration des genetischen Codes die Proteinsequenz SEQ ID NO:2 codieren.

Bevorzugt sind DNA-Vektoren, worin die das Protein Phytoen-Desaturase codierende DNA-Sequenz (a), das *pds*-Gen, aus vorzugsweise *H. pluvialis* stammt und die Resistenz vermittelnde Aminosäuresubstitution durch gerichtete Mutagenese eingeführt wurde. Die erfindungsgemäßen Vektoren codieren für das Protein Phytoen-Desaturase aus *H. pluvialis,* das an Position 504 seiner Aminosäuresequenz einen Aminosäureaustausch von Leucin zu Arginin besitzt (SEQ ID NO: 3).

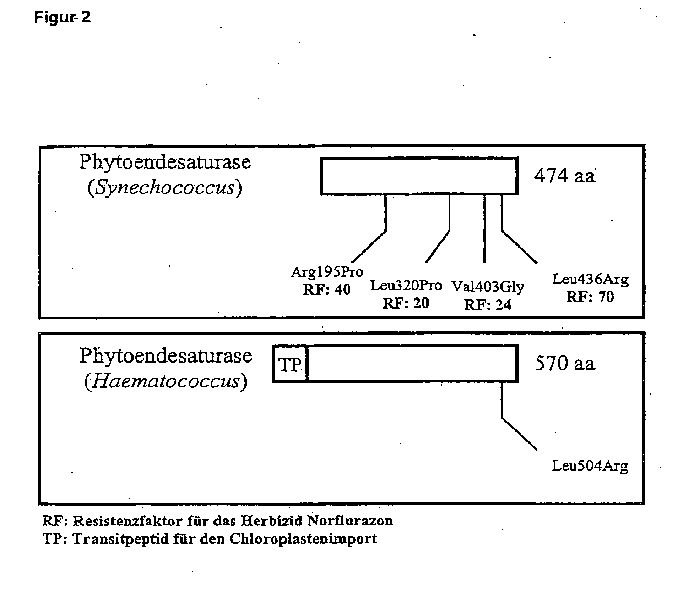

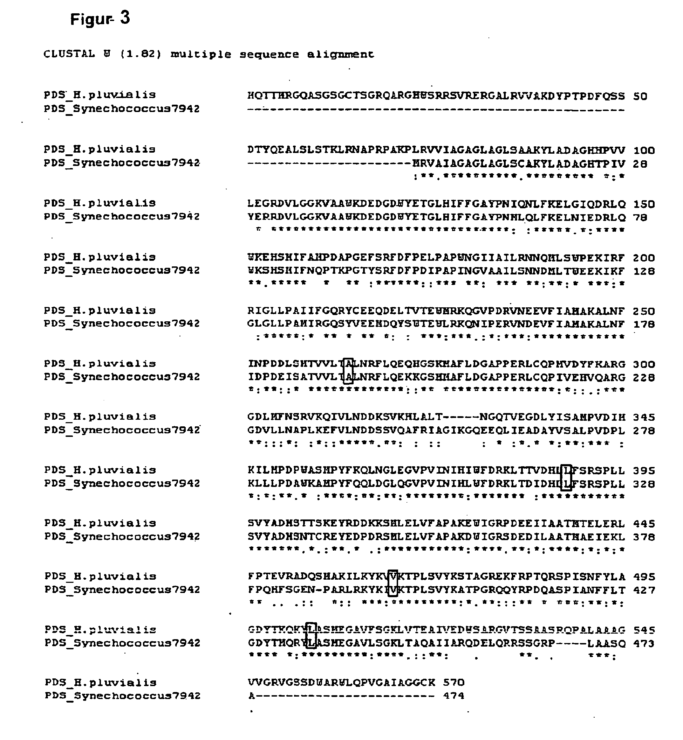

Der erfindungsgemäße Aminosäureaustausch Leucin zu Arginin an Position 504 des PDS-Proteins aus *H. pluvialis* entspricht dem Leu437Arg-Austausch in *Synechococcus PCC7942* (Fig. 2). Durch gerichtete Mutaganese wird hierzu das Leu-Codon "CTG" durch das Arg-Codon "CGC" ersetzt. Diese Mutation verleiht den Mutanten den höchsten bekannten Resistenzfaktor gegenüber Norflurazon. Die vermittelte Resistenz verleiht den erfindungsgemäßen Mutanten im Vergleich zum Wildtyp eine bis zu 70-fach höhere Resistenz gegenüber Norflurazon. Erfindungsgemäß werden Norflurazonkonzentrationen von 0,5 - 50 µM als Selektionssubstanz verwendet.

Weiter beschrieben werden Positionen für einen Aminosäureaustausch in der Aminosäuresequenz von *H. pluvialis,* nämlich Arginin 264, Leucin 388, Valin 465 (Fig. 3). Für den Aminosäureaustausch an homologen Positionen ist in *Synechococcus* eine Resistenz verleihende Wirkung bekannt.

Die erfindungsgemäßen Vektoren vermitteln den Transformanten eine Resistenz gegenüber Herbiziden, insbesondere Bleichherbiziden und besonders bevorzugt Norflurazon.

Im Rahmen der Erfindung wird unter Multiple Cloning Site (MCS), welche auch als Polylinker bezeichnet wird, ein Bereich in einer Nukleinsäuresequenz verstanden, der über eine gehäufte Anzahl verschiedener, benutzbarer Schnittstellen für Restriktionsendonukleasen verfügt, ohne das bei einer stattfinden Restriktionshydrolyse die Funktion anderer Elemente der Nukleinsäuresequenz beieinträchtigt wird. Vorzugsweise wird das DNA-Molekül durch Restriktionsendonukleasen deren Schnittstellen in der MCS festgelegt sind, nur an einer Position hydrolysiert. Beispiele für gebräuchliche MCSs sind dem Fachmann aus kommerziell erhältlichen Vektoren und Plasmiden wohl bekannt. Unter MCS wird im Sinne dieser Erfindung ferner jede Restrikionsschnittstelle innerhalb eines Vektors verstanden, die benutzt werden kann, um beliebige DNA-Sequenzen, gerichtet oder ungerichtet, in die Vektor-Sequenz einzuklonieren, ohne dabei andere erfindungsgemäße Funktionselemente des Vektors zu beeinträchtigen.

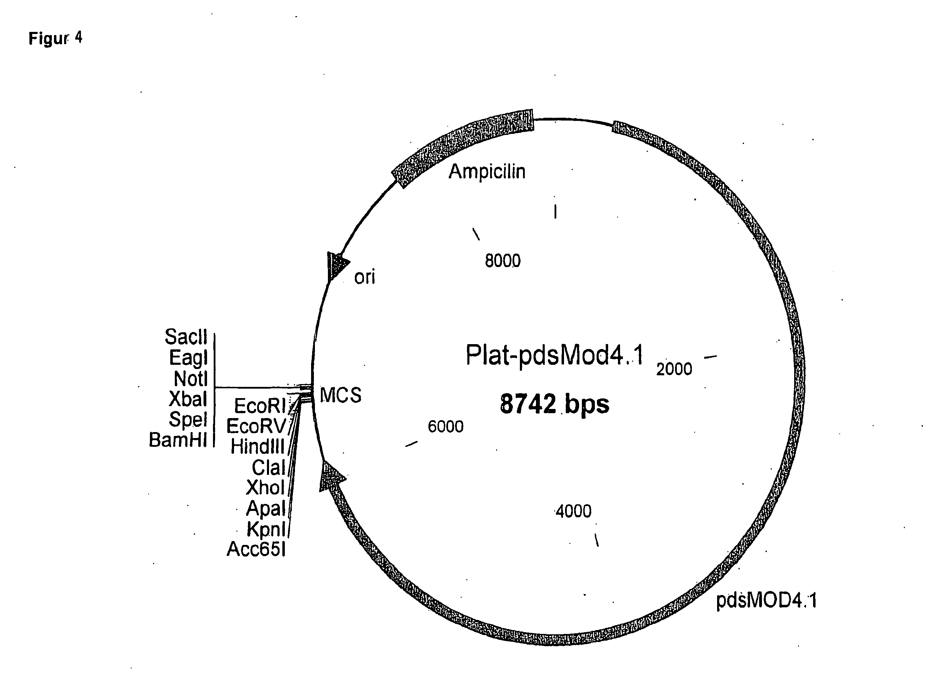

Eine erfindungsgemäß bevorzugte Ausführungsform einer MCS ist in SEQ ID NO: 4 gezeigt. In einer bevorzugten Ausführungsform besitzt der erindungsgemäße DNA-Vektor die Sequenz SEQ ID NO: 5 (s. auch Fig. 4).

Die vorliegende Erfindung betrifft bevorzugt solche DNA-Vektoren, die als einzuklonierende beliebige DNA-Sequenz in der Multiple Cloning Site eine Codiersequenz enthalten.

Unter Codiersequenz wird jede DNA-Sequenz verstanden, die für ein vollständiges aktives Protein oder ein Proteinfragment, welches über biologische Aktivität verfügt, codiert.

Besonders bevorzugt sind Codiersequenzen, die pflanzlicher Herkunft sind. Besonders bevorzugt sind weiterhin Codiersequenzen, die wenigstens eine Promotorsequenz enthalten. Dabei werden Promotoren bevorzugt, die eine konstitutive Transkription bzw. Expression der unter ihrer Kontrolle stehenden Codiersequenzen ermöglichen. Ein bevorzugter Promotor ist der β-Tubulin-Promotor. Insbesondere bevorzugt sind die Promotorsequenzen, die ausgewählt sind aus der Gruppe, die besteht aus *H.pluvialis*-Promotoren des Actin-Gens (SEQ ID NO:6) und des Rubisco-Gens (SEQ ID NO:7).

Weiterhin betrifft die Erfindung DNA-Vektoren, worin die Codiersequenz neben wenigstens einem Promotorgen ein zu exprimierendes Funktionsgen enthält. Besonders bevorzugt sind dabei DNA-Vektoren, die eine Codiersequenz enthalten, die ausgewählt wird aus der Gruppe, die besteht aus Carotinoid-Biosynthesegenen, Astaxanthin-Biosynthesegenen und Isoprenoid-Biosynthesegenen. Insbesondere bevorzugt sind Gensequenzen der β-Carotin-Ketolase, Carotinoid Hydroxylase, ξ-Carotin-Pesaturase, Phytoen-Synthase, Leucopin-Cyclase, Deoxy-Xylulosesynthase und die 1-Deoxy-xylose-5-phosphat-reductosiomerase (Berthold et al., 2002, Hallmann und Sumper, 1996, Mahmoud und Croteau, 2001).

Die Erfindung betrifft weiter die Verwendung des erfindungsgemäßen Vektors zur Transformation von eukaryotischen Zellen, insbesondere einzelligen Pflanzenzellen. Besonders bevorzugt ist die Verwendung des erfindungsgemäßen Vektors zur Transformation von Algenzellen, insbesondere *H.pluvialis*-Zellen. Unter Transformation wird im Rahmen der Erfindung das Einbringen von Fremd-DNA in einen Organismus verstanden.

Die Verwendung des erfindungsgemäßen Vektors als selektiver Marker zur Transformation ist ebenfalls ein Gegenstand der Erfindung. Besonders bevorzugt ist dabei die Verwendung als dominanter selektiver Marker.

Ein selektiver Marker im Sinne der vorliegenden Erfindung vermittelt einem transformierten Organismus eine Eigenschaft, durch welche dieser leicht von nicht transformierten Organismen derselben Spezies zu unterscheiden ist. Unter dominant selektivem Marker wird ein Marker verstanden, der eine Eigenschaft vermittelt, durch welche Selektionsdruck in der Art ausgeübt werden kann, dass in einer Population transformierter und nicht transformierter Organismen derselben Spezies nur transformierte Organismen überlebensfähig sind. Beispielsweise ist es durch die zugabe von Norflurazon zu Wachstumsmedium möglich *H.pluvialis*-Zellen, welche erfolgreich mit dem erfindungsgemäßen Vektor der SEQ ID NO:4 transformiert wurden, von nicht transformierten, unter diesen Bedingungen nicht lebensfähigen Zellen zu selektieren. Die Selektion der Transformanten erfolgt erfindungsgemäß bei Norflurazon-Konzentrationen von 0,5-50 µM. Die Selektion kann in Flüssigkultur oder durch Zugabe des Herbizids zu Nährmediumspiatten erfolgen.

Durch die Verwendung mutierter PDS als erstem dominanten selektiven Marker zur Transformation von Eukaryoten, insbesondere Algen wie *H. pluvialis* leistet die vorliegende Erfindung einen wichtigen Beitrag zur biotechnologischen Nutzung von Eukaryoten, insbesondere Algen wie *H. pluvialis.* Der erfindungsgemäße Vektor kann zum Beispiel mit und ohne Insertion einer Codiersequenz in die MCS verwendet werden, um die Carotinoidbiosynthese in Transformanten zu beeinflussen bzw. zu verändern.

Weiterhin betrifft die Erfindung ein Verfahren zur Transformation von eukaryotischen Zellen unter Verwendung eines erfindungsgemäßen Vektors. Solche Verfahren zur Transformation sind dem Fachmann bekannt. Sie umfassen beispielsweise die Verwendung von PEG, Glasskügelchen, Elektroporation und Mikropartikelbeschuß. Erfindungsgemäß besonders bevorzugt ist ein Verfahren, in welchem die Transformation durch Partikelbeschuss durchgeführt wird. Ein bevorzugte Ausführungsform ist dabei der Partikelbeschuss mit 0,4 bis 1,7 µm großen Wolfram- oder Goldpartikeln, welche zuvor mit efindungsgemäßer Vektor-DNA beschichtet wurden, bei einem Druck von 500 bis 2500 psi und im Vakuum durchgeführt. Nach der Transformation werden die Zellen vorzugsweise in OHA Flüssigmedium (2,42g Tris/Acetat pH6,8) unter Schütteln über Nacht im Dunkeln regeneriert. Die Zellen werden dann unter Selektionsdruck mit 0,7% OHA-Agarose auf OHA-Platten ausplattiert. Transformanten können nach 3-4 Wochen unter Lichtdunkelwechsel von gleichlangem Licht (15-25µE*m^{-2*}s⁻¹) und Dunkelintervallen (je 6-12h) beobachtet werden. Die Transfiormationseffizienz beträgt ca. 1*10⁻⁴ bis 10*10⁻⁸ Zellen/µg DNA, bevorzugt 1*10⁻⁶ Zellen/µg DNA.

Zusätzlich umfasst die Erfindung eine transgene Pflanzenzelle und deren Nachkommen, **dadurch gekennzeichnet, dass** sie einen Vektor nach Anspruch 1 umfasst. Bevorzugt sind dabei transgene Pflanzenzellen und deren Nachkommen, die einen Einfach- oder Mehrfacheinbau der eingebrachten DNA ins nukleäre Genom zeigen. Besonders bevorzugt sind transgene Pflanzenzellen und deren Nachkommen in welchen das eingebrachte Gen konsititutiv exprimiert wird.
- SEQ ID NO:1 :: Nukleinsäuresequenz des pds-Gens, welches für das Protein Phytoen-Desaturase aus *H. Pluvialis* codiert
- SEQ ID NO:2 :: Proteinsequenz der Phytoen-Desaturase aus *H. pluvialis*
- SEQ ID NO:3 :: Proteinsequenz der Phytoen-Desaturase mit Leu504 Arg Aminosäureaustausch
- SEQ ID NO:4 :: Nukleinsäuresequenz der MCS des erfindungsgemäßen Vektors Plat-pdsMod4.1
- SEQ ID NO:5 :: Nukleinsäuresequenz des bevorzugten DNA-Vektors Plat-pdsMOD4.1
- SEQ ID NO:6:: Nukleinsäuresequenz umfassend den Actin-Promotor (Smal-Fragment). Die Nukleinsäuresequenz umfassst codierende Bereiche mit Introns, Exons und die Promotorsequenz, welche hervorgehoben (-) ist.
- SEQ ID NO:7 :: Nukleinsäuresequenz umfassend den rbsc-Promotor (Rubisco small subunit) (Pstl-Fragment). Die Nukleinsäuresequenz umfasst codierende Bereiche mit Introns, Exons und die Promotorsequenz, welche hervorgehoben ist.

- Fig. 1:: Carotinidbiosyntheseweg von Geranyl-Geranyl-Diphosphat bis Astaxanthin
- Fig. 2:: Gegenüberstellung von Phytoen-Desaturase aus *Synechococcus* und *H. pluvialis* mit bekannten Mutationen und Resistenzfaktoren (RF) gegenüber Norflurazon
- Fig. 3:: Aminosäuresequenzalignment der PDS-Proteine aus *H. pluvialis* und *Synechococcus.* Für eine Resistenz gegenüber Norflurazon verleihenden Aminosäureaustausch bevorzugte Positionen sind markiert.
- Fig. 4:: Plasmidkarte des Vektors Plat-pdsMod4.1; MCS (Multiple cloning site), ori (origin of replication)
- Fig. 5:: Southernblot genomischer DNA nach Xbal und Xhol Verdau und Nachweis über Phytoen-Desaturasesonde. Marker (M) in Kilobasen (kb)
- Fig. 6:: (A) Northernblot von drei unter Dauerstarklichtstress (120 µE*m⁻²*s⁻¹)- gesetzten WT Stämmen (WT 0, 18 und 36 Stunden) und den Transformanten P1 - P13. (B) Westernblot mit Antikörper gegen
- Fig. 7:: das Phytoen-Desaturase Protein. Die Bande läuft auf Höhe der berechneten 55 kDa. Phytoen-Desaturase mRNA (*pds*), Carotinoid Hydroxylase mRNA (*hyd*), Phytoen-Desaturase Protein (PDS) Darstellung des Non- photochemical quenching (NPQ). NPQ = (F° ₘ-F'ₘ)/F'ₘ F°ₘ ist die maximale Fluoreszenz dunkeladaptierter Organismen nach einem sättigenden Lichtpuls. F'ₘ maximale Fluoreszenz nach sättigenden Lichtpulsen in definierten Intervallen bei 20 s.
- Fig. 8:: Darstellung der Akkumulation des Ketocarotinoids Astaxanthin im WT *H. pluvialis* und zweier Transformanten (P3, P13) ohne Herbizidzugabe unter Dauerstarklicht (175 µEm⁻²s⁻¹). Trockengewicht (DW), Zeit in Stunden (h).
- Fig 9:: Transformationsvektor zur konstitutiven Expression des Hydroxylasegens (hyd) unter der Kontrolle des Promotors der kleinen Untereinheit der Rubisco (rbcS2)

### Beispiele:

### Herstellung des Transformatlonsvoktors zur Transformation von H. pluvialis

Ein 6,1 Kilobasen großes Stück der Phytoen-Desaturase wurde aus einer genomischen DNA-Bibtiothek von *H. pluvialis* isoliert und über die Restriktionsschnittstellen Xbal und Xhol in die Multiple Cloning Site (MCS) des pBluescriptSK-Vektor (Stratagene) subkioniert. Über eine gerichtete Mutagenese wurde mit den Primern PDS-Qmut-plu2 CCA AGC AGA AGT ACC GCG CCT CCA TGG AGG G und PDS-Qmut-min2 CCC TCC ATG GAG GCG CGG TAC TTC TGC TTG G ein Nukleotidaustausch von CTG in CGC durchgeführt, das Plasmid wurde als pPDS-Q2 bezeichnet. Dieser Nukleotidaustausch führt zu einem Aminosäureaustausch von Leucin zu Arginin in Codon 504 (Fig. 2), und vermittelt Mutanten eine Resistenz gegenüber Norflurazon.

Von dem Plasmid pPDS-Q2 ausgehend wurden durch PCR mit den spezifischen Primem die endständigen Restriktionsschnittstellen Xbal und XhoI eliminiert und zwei neue EcoRV Schnittstellen eingeführt. Über diese Restriktionsschnittstellen wurde das mutierte Phytoen-Desaturasegen in eine Nael Schnittstelle eines pBluescriptSK- Vektors subkloniert. Die Multiple Cloning Site (SEQ ID NO: 4) des Vektors ist dadurch frei für andere Klonierungen. Das so hergestellte Transformationsplatform wurde als Plat-pdsMod4.1 bezeichnet (Fig. 4).

### Transformation von H. pluvialis mit dem Transformationsvektor Plat-pdsMod4.1

*H. pluvialis* Zellen wurden für 4 Tage in Flüssigmedium unter Standardbedingungen (Lichtdunkelwechsel von 12 Stunden Licht (20µE*m^{-2*}s⁻¹)und 12 Stunden Dunkelheit) bis zu einer Zelldichte von 3,5*10⁵ Zellen/ml angezogen (Kobayashi, Kakizono et al. 1991). Die Zellen wurden für 5 min bei 16 °C und 4000 x g abzentrifugiert, resuspendiert und jeweils 1*10⁸ Zellen auf Nylonfilter (Roche) ausplattiert. Die Filter wurden auf OHM Mediumsplatten (Fabregas, Dominguez et al. 2000) überführt und bis zur Transformation getrocknet. Die 0,4-1,7 µm großen Wolfram Partikel wurden mit 2µg Plat-pdsMod4.1 Vektor DNA nach dem Protokoll von Klein und Mitarbeitern beschichtet (Klein, Wolf et al. 1987). Transformiert wurde mit der Partikelgun PDS-1000/He der Firma Bio-Rad bei einem Druck von 1350 psi und einem Vakuum von 25 mmHg. Ansonsten wurden die Standardeinstellungen beibehalten.

Nach der Transformation wurden die Zellen auf den Nylonfilter in OHA Flüssigmedium (2,42 g Tris/Acetat pH 6,8) über Nacht unter leichtem Schütteln im Dunkeln regeneriert. Danach wurden die Zellen kurz abzentrifugiert und mit 0,7 % OHA Top-Agarose auf 10 bis 20 OHA Platten (5 µM Norflurazon) ausplattiert. Die ersten Transformanten konnten nach einer Wachstumsphase von drei bis vier Wochen unter einem Lichtdunkelwechsel von 12 Stunden Licht (20 µE*m⁻²*s⁻¹) und 12 Stunden Dunkelheit beobachtet werden. Die Transformationseffizienz liegt etwa bei 1*10⁻⁶ Zellen/µg DNA.

### Molekulare Analysen der mit dem Vektor Plat-pdsMod4.1 transformierten Zellen

Die positiven Transformanten wurden mehrfach auf OHA Platten mit einer Norflurazonkonzentration von 0,7 µM überimpft. Das Wachstum der WT Zellen ist bei diesen Konzentrationen stark gehemmt. Zur molekularen Untersuchung der Transformanten über Southem- Northern- und Westemblotanalysen wurden die Transformanten in Flüssigmedium mit einer Norflurazonkonentration von 3 µM angezogen.

### Southernblot, Kontrolle der Integration des Transformationsplasmids im Genom von H. pluvialis

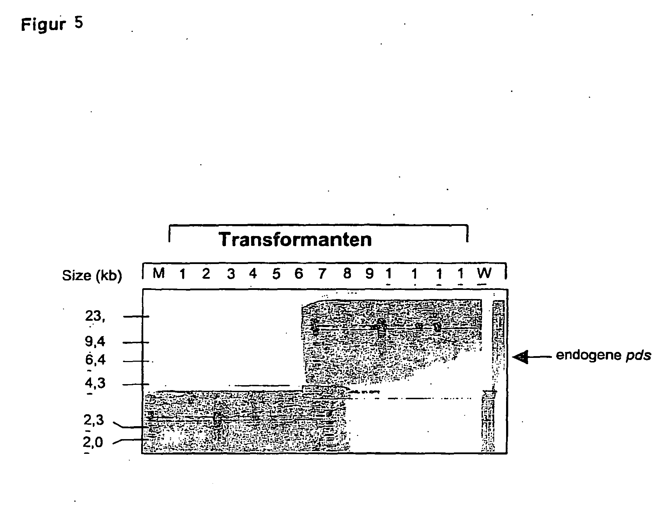

Nach einem Wachstum von vier Tagen unter Standardbedingungen bei einer' Norflurazonkonentration von 3 µM in Flüssigmedium, wurden die Zellen abzentrifugiert und die genomische DNA aus den verschiedenen Transformanten, sowie mehreren WT Kontrollen isoliert. Die gewonnene genomische DNA wurde mit den Restriktionsenzymenen XbaI und XhoI verdaut und auf einem 0,8%igem Agarosegel aufgetrennt. Die DNA wurde nach Standardmethoden geblottet und mit einer Sonde gegen die PDS hybridisiert (Fig. 5).

In Southemblot läßt sich deutlich die endogene Phytoen-Desaturase bei etwa 5,9 kb in allen Transformanten, wie auch im WT wiederfinden. Die zusätzlich integrierten mutierten Phytoen-Desaturasen laufen in allen Fällen höher als die endogene Phytoen-Desaturase. Von anderen Organismen, wie etwa dem Pilz *Neurospora crassa* ist bekannt, daß Vektoren sich häufig als tandem repeats in das Genom integrieren (Cogoni and Macino 1997). Dieses Phänomen erklärt die sehr starken Banden in Transformanten P6, 7, 11 und P13. Die genomische DNA wurde zusätzlich noch mit einer Sonde gegen die Ampicilin Resistenzkasette des Transformationsvektors PlatpdsMod4.1 hybridisiert. Unter diesen Bedingungen waren in allen Fällen, außer bei der Transformante P3, mehrere deutliche Banden zu erkennen.

### Northern- und Westernblot Analysen der Transformanten P1 - P13

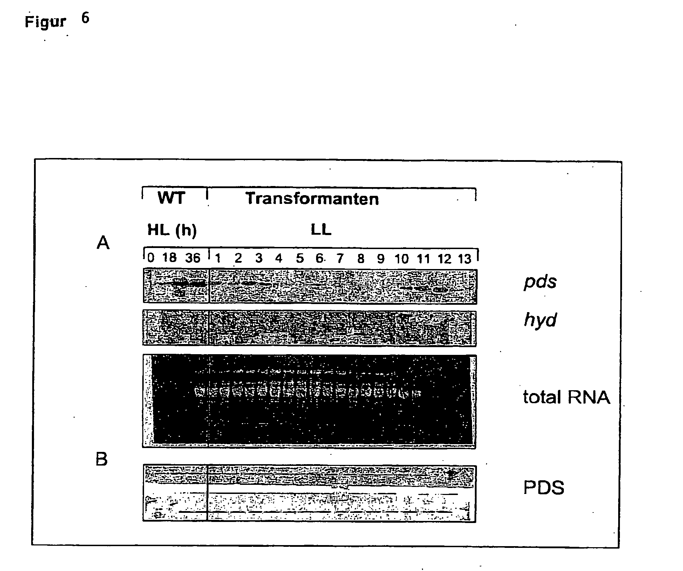

Nach einem Wachstum von vier Tagen unter Standardbedingungen bei einer Norflurazonkonentration von 3µM in Flüssigmedium, wurden die Zellen abzentrifugiert und die RNA aus den verschiedenen Transformanten, sowie mehreren WT-Kontrollen isoliert. Für die weitere Analyse der Transkriptionsmuster der Transformanten wurden die RNA Proben auf einem 1 %igen denaturierenden Agarosegel aufgetrennt, geblottet und mit einer Sonde gegen die Phytoen-Desaturase mRNA (*pds*) und gegen die Carotinoid Hydroxylase mRNA (*hyd*) hybridisiert (Fig. 6A). Die Hydroxylase dient als interner Standard, um Aussagen über den Stresszustand der Transformanten machen zu können. Die Hydroxylase wird unter Stressbedingungen wie Licht- oder Salzstress induziert und liegt sonst unter der Nachweisgrenze (Steinbrenner and Linden 2001; Steinbrenner and Linden 2003).

Für die Gewinnung von Proteinproben wurden die Zellen ebenfalls nach vier Tagen Wachstum unter Standardbedingungen geerntet und auf einem 12,5 . %igen SDS Polyacrylamidgel getrennt. Das Phytoen-Desaturase-Protein wurde mit einem spezifischen Antikörper nachgewiesen (Grunewald, Eckert et al. 2000).

Die unter Dauerstarklichtbedingungen (120 µE*m^{-2*}s⁻¹) gesetzten WT Zellen zeigen für die Phytoen-Desaturase (*pds*) eine basale Expression des pds Gens zum Zeitpunkt der Induktion (0 Stunden). Dieses Signal nimmt im Verlauf über 18 Stunden zu. Die Carotinoid Hydroxylase verhält sich unter diesen Induktionsbedingungen adequat, die Transkription ist aber etwas verzögert.

Die Expressionsmuster der Phytoen-Desaturase mRNA der Transformanten P1, P2, P5, und P6 sind mit der Basalexpression des WT bei 0 Stunden unter Standardbedingungen zu vergleichen. Die Expressionslevel für die Transformanten P8, P9 und P10 liegen sogar noch etwas unter dem Level der Basalexpression des WT bei 0 Stunden. In zwei weiteren unabhänigen Northemblotanalysen entsprach die Expression der Transformanten P8, P9 und P10 der Basalexpression des WT bei 0 Stunden. Die Transformanten P3, P4, P7, P11, P12 und P13 zeigen hier einen erhöhten Transkriptionslevel, der im Vergleich mit der Dauerstarklichtkontrolle zwischen 0 Stunden und 18 Stunden liegt; dies wurde in zwei weitem unabhänigen Northemblotanalysen ebenfalls beobachtet. Die Erhöhung ist in diesem Fall nicht auf eine Induktion durch Stress zurüchzuführen, sondern auf einen Mehrfacheinbau des Transformationsvektors Plat-pdsMod4.1 in den Genom von *H. pluvialis.* Die Expression der Carotinoid Hydroxylase als Stresskontrolle zeigt unter diesen Standardbedingungen keine erhöhten Transkriptionsievel.

Im Westernblot der mit einem Antikörper gegen die PDS nimmt die Proteinmenge bis zum 36 Stunden Wert stetig zu (Fig. 6B).

Die Proteinmengen aller Tranformanten liegen leicht über dem 0 Stunden Wert der WT Kontrolle. Die Proteinmengen der Transformanten P3, P5, P7, P11 und P13 sind gegenüber den anderen Leveln nochmals erhöht und mit der Proteinmenge des WT 18 Stunden Wertes unter Dauerstarklichtbedingungen zu vergleichen. Die stärkeren Transkriptionslevels der Phytoen-Desaturase lassen sich auch auf Proteinebene in den Transformanten P3, P7, P11 und P13 wiederfinden. **Physiologische Untersuchungen der Plat-pdsMod4.1 Transformanten**

### Bestimmung des Non photochemical quenchings (NPQ) der Plat-pdsMod4.1 Transformanten

Einige höhere Pflanzen mit veränderter Carotinoidbiosynthese wurden in der Vergangenheit näher untersucht So fand eine Transformation von Tabak mit dem Phytoen-Desaturase-Gen *crtl* aus *Erwinia uredovora* statt (Misawa, Yamano et al. 1993). In einem der resultierenden Transformanten, mit ET4-208 bezeichnet, wurde eine höhere Resistenz gegenüber Norflurazon festgestellt. Später konnte auch eine veränderte Carotinoidzusammensetzung in den transgenen *crtl*-Pflanzen gegenüber nicht transformierten Kontrollen nachgewiesen werden (Misawa, Masamoto et al. 1994).

Eine Möglichkeit Veränderungen der Carotinoidzusammensetzung in transgene Pflanzen zu untersuchen, ist die Messung der Chlorophyllfluoreszenz. Einerseits lassen sich so sehr schnell Photosynthese Mutanten screenen, andererseits spiegelt sich eine Veränderung der Xanthophyllgehalte in der Chlorophyll- Fluoreszenz wieder (Niyogi, Bjorkman et al. 1997).

Die verschiedenen Transformanten wurden auf OHA Platten ohne Norflurazon unter Standardbedingungen für drei bis vier Wochen angezogen. Vor den Messungen wurden die Platten für 24 Stunden dunkeladaptiert. Unter Verwendung eines PAM-Fluorimeter (Walz, Deutschland) wurde von allen Transformanten die Chlorophyllfluoreszenz gemessen und das NPQ über folgende Formel berechnet: NPQ = (F°ₘ-F'ₘ)/ F'ₘ

F°ₘ ist die maximale Fluoreszenz dunkeladaptierter Organismen nach einem sättigenden Lichtpuls. F'ₘ maximale Fluoreszenz nach sättigenden Lichtpulsen in definierten Intervallen; in unserem Fall lagen die Intervalle bei 20 s. Ein guter Übersichtsartikel zu diesem Thema wurde von Kate Maxwell und Giles N. Johnson verfasst (Maxwell and Johnson 2000).

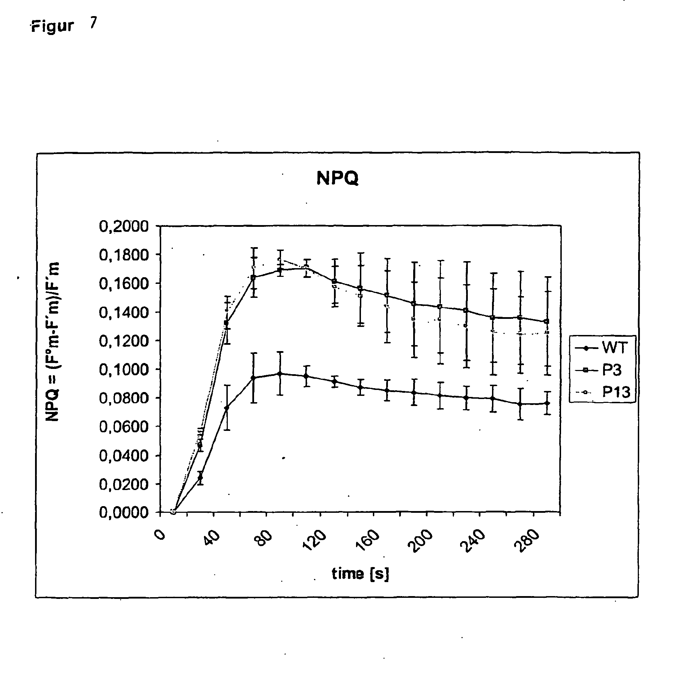

Die gemessenen Transformanten zeigten alle, bis auf Ausnahme von P3 und P13 Fluoreszenzkurven und NPQ-Kurven wie der Wildtyp (WT). Das NPQ der Transformanten P3 und P13 liegt im ganzen Messverlauf etwa 50 % höher als bei dem WT (Fig. 7).

### Vergleich der Verteilung der gefärbten Carotinoide im WT H. pluvialis und dreier Transformanten ohne Herbizidzugabe

Die Transformanten wurden unter Standardbedingungen in Flüssigmedium ohne Herbizidzugabe für vier Tage angezogen, die Zellen abzentrifugiert und die Pellets gefriergetrocknet. Die Trockengewichte der Pellets wurden bestimmt und die Zellen unter Zugabe von Methanol gemörsert. Die Chlorophylle wurden photometrisch gemessen und dann später unter Zugabe von 6% KOH verseift. Die Carotinoide wurden in Petrolether/Ether (Sp 35 °C -60 °C) (9:1 v/v) gezogen, eingedampft und in 100 %igen Aceton aufgenommen. Die Proben wurden dann über eine HPLC Säule aufgetrennt.

**Tab. 1: HPLC-Analyse der Gesamtpigmentextrakte von H. pluvialis WT und den Phytoen-Desaturase Transformante P1, P3 und P13. Prozentuale Verteilung der Carotinoide am Gesamtcarotinoid.**

| | *H. pluvialis* **WT** | Transformante **P1** | Transformante **P3** | Transformant **e P13** |
|---|---|---|---|---|
| Neoxanthin | 9,6 | 9,6 | 12,9 | 8,9 |
| Violaxanthin | 8,6 | 8,3 | 11,7 | 11,23 |
| Lutein | 65,3 | 62,9 | 65,8 | 64 |
| β -Carotin | 16,6 | 19 | 9,6 | 15,7 |

Wie in Tabelle 1 gut ersichtlich, ist die prozentuale Verteilung der einziehen Carotinoide im WT und in der Transformante P1 sehr ähnlich. Im Vergleich dazu haben sich die Carotinoidzusammensetzungen in Transformanten P3 und P13 stark verändert. In P3 sind die Xanthophylle etwa um 6% erhöht und der β-Carotingehalt im Vergleich mit dem WT um diesen Wert verringert In der Transformante P13 ist der Violaxanthingehalt um etwa 3% erhöht. Zeaxanthin welches direkt am NPQ Prozess beteiligt ist, kann unter Standardbedingungen nicht gemessen werden Hierfür wären HPLC Messungen von starklichtgestressten Zellen nötig. Die Erhöhung der Violaxanthingehalte um etwa 3% der Transformanten P3 und P13 läßt trotzdem eine direkte Kopplung zum erhöhten NPQ dieser Transformanten zu, da der Violaxanthinpool unter Starklichtbedingungen in Zeaxanthin umgewandelt wird.

### Carotinoidzusammensetzung der H. Pluvialis Transformanten P3, P13 und WT unter Schwachlichtbedingungen.

Es ist bekannt, dass die heterologe Expression des pds-Gens aus *Erwinia uredovora* in Tabak die Zusammensetzung der Carotinoide im Blatt verändert (Misawa et al.. (1994)). Die Analyse Norflurazon-resistenter Synechococcus-Mutanten bestätigte, dass die Phytoendesaturierung bei der Carotinoid-Biosynthese der geschwindigkeitsbestimmende Schritt in Cyanobakterien ist (Chamovitz et al., (1993)). Zur Bestätigung dieser Ergebnisse wurden die P3, P13 und WT *H. pluvialis* Transformanten unter Schwachlichtbedingungen aufgezogen und alle Carotinoide extrahiert. Keine der untersuchten Transformanten zeigte im Vergleich zum Trockengewicht veränderte Carotinoidmengen. Eine anschließend durchgeführte HPLC-Analyse der Carotinoidzusammensetzung in den Transformanten P3, P13 und WT zeigte für die Transformante P3 eine leicht erhöhte Menge des Xanthophylls Violaxanthin, wohingegen die Mengen an Lüthein und Neoxanthin leicht verringert waren.

### Vergleich der Akkumulation des Ketocarotinoids Astaxanthin im WT H. pluvialis und der Transformanten P3, und P13 ohne Herbizidzugabe unter Dauerstarklicht

Für die Starklichtexperimente wurden die *H. pluvialis-*Zellen für 4 Tage unter Standardbedingungen angezogen. Nach dieser Periode wurde die Lichtstärke von 20 µEm⁻²s⁻¹ auf 175 µEm⁻²s⁻¹ und der Licht/Dunkelrhythmus auf 24 Stunden Dauerlicht erhöht. Zu bestimmten Zeiten (0, 6, 12, 24, 48, 72 Stunden) nach Induktion wurden Proben genommen und abzentrifugiert. Die Carotinoid-Extraktion wurde nach der Methode von Boussiba *et al*. (1992) ausgeführt. So wurde das Trockengewicht (TG) der gefriergetrockneten Zellen bestimmt und unter Zugabe von Seesand, 30% Methanol und 5% KOH nochmals gemörsert. Nach der Verseifung der Chlorophylle für 10 min bei 70°C wurden die Proben für 5 min bei 4000 U/min abzentrifugiert und der chlorophyllhaltige Überstand verworfen. Das Pellet wurde in 100% DMSO (Dimethylsulfoxid) aufgenommen und für 10 min auf 70°C erhitzt. Dieser Extraktionsschritt wurde so oft wiederholt, bis das Pellet keine rötliche Färbung mehr zeigte. Astaxanthin und seine Ester zeigen ihr Hauptabsorptionsmaximum bei 492 nm. Auf eine Messung bei dieser Wellenlänge wurde aber verzichtet, da die Gesamtcarotinoide (hauptsächlich β-Carotin, Lutein und Violaxanthin) auch eine Absorption in diesem Wellenlängenbereich zeigten. So wurde unter Zuhilfenahme eines Astaxanthinstandards (Sigma, in 100% DMSO) ein Berechnungsfaktor bestimmt, der es erlaubte den Astaxanthingehalt in einem Wellenlängenbereich (550 nm) zu bestimmen, bei dem die Gesamtcarotinoide keine Absorbtion mehr zeigten. So wurde die Absorption des Astaxanthinstandards erst bei einer Wellenlänge von 492 nm (A₄₉₂) und anschließend bei einer Wellenlänge von 550 nm (A₅₅₀) gemessen. Mit dem Quotienten A₄₉₂/A₅₅₀ = 3.2 wurden dann die bei 550 nm gemessenen Absorbtionswerte multipliziert und der Astaxanthingehalt mit einem Absorptionskoeffizienten für Astaxanthin (E1%/1cm = 2220) nach der Formel von Davies (1976) berechnet.

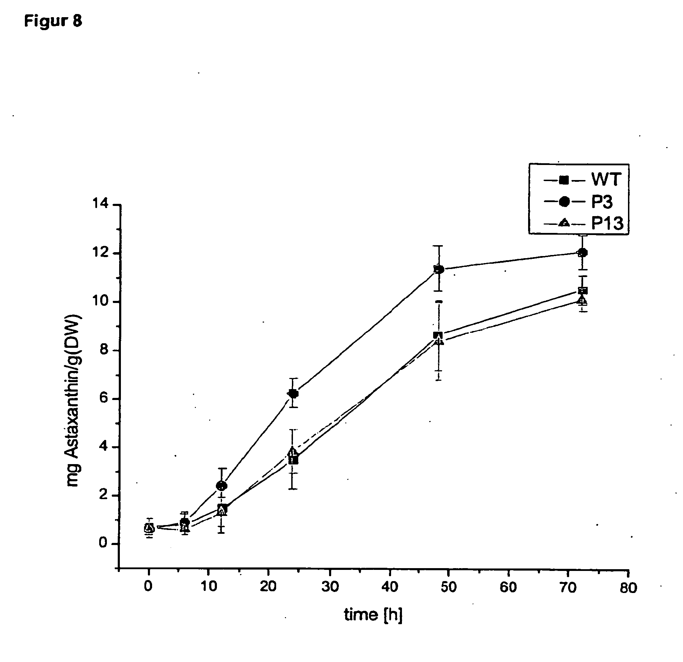

Sechs Stunden nach Änderung der Lichtbedingungen war noch kein Astaxanthin detektierbar (Fig. 8). Nach 8 h Exposition gegenüber Starklicht zeigte die Transformante P3 einen sichtbar roten Phänotyp, während der WT und die anderen Transformanten grün blieben. 12 Stunden nach Induktion lag die Menge an gebildeten Astaxanthin im WT und der Transformante P13 bei 1,47 mg/gTG und 1,32 mg/gTG, im Gegensatz dazu lag der erreichte Wert für die Transformante P3 bei 2,37 mg/gTG. Nach 24 Stunden lag die gemessene Astaxanthinmenge des WT bzw. der

Transformante P13 bei 3,48 mg/gTG bzw. 3,79 mg/gTG. Die Transformante P3 zeigte zu diesem Zeitpunkt mit einer Astaxanthinmenge von 6,22 mg/gTG relativ zum Wildtyp eine etwa 40 %ige Erhöhung. Nach 48 Stunden unter Dauerstarkticht zeigte der WT bzw. die P13-Transformante Werte von 8,6 ± 1,4 mg/gTG bzw. 8,4 ± 1,6 mg/g TG. Eine im Vergleich zu WT bzw. P3 um 26 % erhöhte Astaxanthinakkumulation konnte für die Transformante P3 nach 48 Stunden bestimmt werden (11,4 ± 0,9 mg/g TG). 72 Stunden nach Dauerlichtstress zeigte der WT bzw. die P13-Transformante eine Menge von 10,5 mg/gTG und 10,1 mg/gTG, wobei die Transformante P3 mit einem Wert von 12,1 mg/gTG eine um 14 % höhere Akkumulation von Astaxanthin zeigte.

Die Transformante P3 unterschied sich somit stark hinsichtlich ihrer Akkumulation von Astaxanthin unter Dauerstarklicht im Vergleich zu der Transformante P13 und dem WT. Im Verlauf des Starklichtexperimentes zeigte P3 im Vergleich mit dem WT eine Erhöhung der Astaxanmthinmengen um etwa 40 % nach 24 Stunden. Dieser Unterschied nimmt nach 48 Stunden auf 26 % ab und erreicht nach 72 Stunden einen Wert von 14 %.

Die Ergebnisse zeigen, dass in *H. pluvialis* der Phetoindesaturierungsschritt unter Starklichtbedingungen geschwindigkeitsbestimmend ist, nicht jedoch unter Schwachlichtbedingungen.

### Steigerung der Astaxanthinbiosynthese durch die Verwendung von β-Carotin Ketolase unter der Kontrolle von konstitutiven Promotoren

### Konstruktion und Transformation der Vektoren

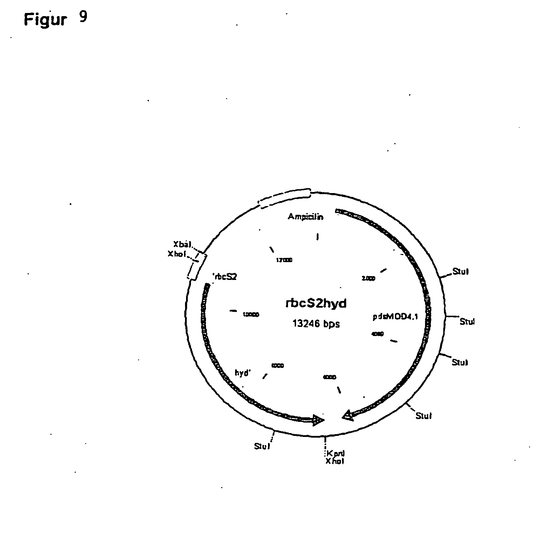

Unter der Verwendung der Transformationsplattform Plat-pdsMod4.1 wurden Transformationsplasmide mit Promotoren des Actingens oder der Ribulosebisphosphat-Carboxylase mit darauf folgender Klonierungsstelle für verschiedene Restriktionsendonukleasen hergestellt, beide Promotoren wurden aus einer genomischen DNA-Bibliothek isoliert. Diese Plattformen enthalten weiter den 3'nicht-translatierten Bereich der jeweiligen Gene, welche auch als Polyadenylierungssignal für eingebrachte cDNAs dienen. Über PCR wurden die cDNAs der Carotinoid-Hydroxylase und der β-Carotin-Ketolase amplifiziert und mit endständigen Restriktionsschnittstellen versehen. Dadurch entstand die Möglichkeit diese cDNAs im Leseraster in die Transformationsplattform einzubringen. Die jeweiligen Konstrukte wurden auch für genomische DNA der beiden Carotinoidbiosynthesegenen hergestellt. In Fig. 9 ist ein Transformationsvektor für die konstitutive Expression des Hydroxylasegens unter der Kontrolle des Promotors der kleinen Untereinheit der Rubisco dargestellt.

Es wurden für die cDNAs und die genomischen Sequenzen der Hydroxylase drei Konstrukte unter der Kontrolle eines Actin-Promotors mit unterschiedlichen Translationsstarts und je ein Konstrukt unter der Kontrolle des Rubisco-Promotors kloniert und in *H. pluvialis* transformiert. Der Selektionsdruck wurden durch eine Verringerung der Norflurazonkonzentration von 5 µM auf 3 µM gesenkt. Dadaurch sollten auch Transformanten erhalten werden, die einerseits eine geringere Resistenz gegenüber Norflurazon besitzen und deren Wachstumschanchen andererseits aufgrund einer Verschiebung des metabolischen Gleichgewichts Richtung Zeaxanthin oder Canthaxanthin verschlechtert wurden. Eine Verschiebung dieses Gleichgewichts könnte beispielsweise eine herabgesetzte Bildung von Lutein bedeuten, welches bei der Zusammenlagerung von Antennenkomplexen in der Thylakoidmembran eine wichtige Rolle besitzt. Die Bildung der Chlorophylle oder auch die Bildung von Tocopherolen könnten durch eine solche Verschiebung ebenfalls betroffen sein, da sich diese ebenfalls von der sehr frühen Isoprenoidbiosynthese abspalten.

### Selektion der Transformanten

Nach der Transformation wurden die erhaltenen Kolonien auf weitere Nährmediumsplatten mit Norflurazon überimpft. Danach wurden von jedem Konstrukt 15 Transformanten in Flüssigmedium unter sehr schwachen selektiven Druck überimpft.

Nach einer weitem Wachstumsphase, bei der keine der angeimpften Tansformanten eine deutliche Carotinoidakkumulation von Canthaxanthin oder Zeaxanthin zeigte, wurden die Zellen für 24 Stunden unter Starklicht (120µE*m⁻²*s⁻¹) gesetzt.

Die Transformanten, die mit den Hydroxylase Konstrukten aus genomischer DNA unter der Kontrolle des Rubisco-Promotors (Plat-rbcS2gHyd) und des zweiten Actin-Promotors (Plat-ActPII-gHyd) (zweiter Translationsstart mit einem Intron) transformiert wurden, zeigten im Vergleich mit den Transformanten der anderen Hydroxylase Konstrukte mehr rot-bräunlich gefärbte Zellen.

Bei den Transformanten der Ketolase Konstrukte, wurden diejenigen weiteruntersucht, die mit der cDNA und dem Ketolasegen unter der Kontrolle des zweiten Actin-Promotors (Plat-ActPllgBkt und Plat-ActPllcBkt) (zweiter Translationsstart mit einem Intron) transformiert wurden.

### Southernblot und Northernblot Analysen der Hydroxylase Konstrukte

Von den jeweils 15 vorgescreenten Transformanten wurden 10 ausgewählt und weiter für molekularbiologische Untersuchungen angezogen. Nach Isolation der genomischen DNA der einzelnen Transformanten wurden diese restriktionsverdaut und auf einem Agarosegel aufgetrennt, geblottet und mit einer Sonde gegen die Hydroxylase hybridisiert. Von den 10 Transformanten, die unter der Kontrolle des zweiten Actinspromotors (Plat-ActPII-gHyd) standen, zeigten zwei Transformanten eindeutig zusätzliche Kopien der Hydroxylase im Genom. Von 10 Transformanten unter der Kontrolle des Rubisco-Promotors (Plat-rbcS2gHyd) waren 6 mit zusätzlichen Kopien im Genom positiv. Die Verringerung der Norflurazonkonzentration auf den Selektionsplatten zugunsten von wachstumsbenachteiligten Transformanten wirkt sich auf die Selektion eindeutig negativ aus.

Von den verbleibenden Transformanten wurde die RNA extrahiert und auf einem denaturienden Agarosegel aufgetrennt, geblottet und mit einer Sonde gegen die Hydroxylase mRNA hybridisiert. Von den Transformanten zeigten zwei einen erhöhten Transkriptlevel der mRNA der Hydroxylase. Kontrollexperimente mit anderen Sonden gegen Carotinoidbiosynthesegene β-Lycopincyclase und Phytoensynthase zeigten bei diesen beiden Transformanten ebenfalls einen erhöhten Transkriptlevel.

### Literatur:

1. Teng, C., Qin, S., Liu J., Yu, D:, Liang C. und Tseng, C. 2002. Transient expression of lacZ in bombarded unicellular green alga Haematococcus pluvialis. Journal of Applied Phycology 14: 495-500.
2. Boussiba, S. und Vonshak, A. 1991. Astaxanthin accumulation in the green alga Haematococcus pluvialis. Plant Cell Physiol. 32: 1077-1082.
3. Fraser, P.D., Shimada, H. und Misawa, N. 1998. Enzymic confirmation of reactions involved in routes to astaxanthin formation, elucidated using a direct substrate in vitro assay. Eur. J. Biochem. 252: 229-236.
4. Hawkins, R.L. und Nakamura, M. 1999. Expression of human growth hormone by the eukaryotic alga, Chlorella. Curr. Microbiol. 38: 335-341.
5. Jyonouchi, H., Sun, S. und Gross, M. 1995. Effect of carotenoids on in vitro immunoglobulin production by human peripheral blood mononuclear cells; astaxanthin, a carotenoid without vitamin A activity, enhances in vitro immunoglobulin production in response to a T-dependent stimulant and antigen. Nutr. Cancer 23: 171-183.
6. Kim, D.H., Kim, Y.T., Cho, J.J., Bae, J.H. und Hur, S.B. 2002. Stable integration and functional expression of flounder growth hormone gene in transformed micro alga Chlorella ellipsoidea. Marine Biotech. 4:63-73.
7. Kindle, K.L. 1990. High-frequency nuclear transformation of Chlamydomonas reinhardii. Proc. Natl. Acad. Sci. USA 87: 1228-1232.
8. Kobayashi, M. und Sakamato, Y. 1999. Singlet oxygen quenching ability of astaxanthin esters from the green alga Haematococcus pluvialis. Biotech. Lett. 21: 265-269.
9. Lu, E., Vonshak, A., Gubbay, R., Hirschberg, J. und Boussiba, S. 1995. The biosynthetic pathway of astaxanthin in a green alga Haematococcus pluvialis as induced by inhibition with diphenylamine. Plant Cell Physiol. 36: 1519-1524.
10.Lumbreras, V., Stevens, D.R. und Purton, S. 1998. Efficient foreign gene expression in Chlamydomonas reinhardii mediated by an endogenous intron. Plant J. 14: 441-447.
11.Tanaka, T., Makita, H., Ohnishi, M., Mori, H., Satoh, K. und Hara, A. 1995. Chemoprevention of mouse urinary bladder carcinogenesis by the naturally occurring carotenoids astaxanthin. Carcinogenesis 15: 15-19.
12.Chamovitz, D., Sandmann, G. und Hirschberg, J. 1993. Molecular and biochemical characterization of herbicide-resistant mutants of cyanobacteria reveals that phytoene desaturation is a rate-limiting step in carotenoid biosynthesis. The Journal of Biological Chemistry, vol. 268, no. 23, S. 17348-17353.
13.Martinez-Férez, I., Vioque, A. und Sandmann, G. 1994. Mutagenesis of an amino acid responsible in phytoene desaturase from Synechocystis for binding of the bleaching herbicide norflurazon. Pesticide Biochemistry and Physiology 48: 185-190.
14.Martinez-Férez, I.M. und Vioque, A. 1992. Nucleotide sequence of the phytoene desaturase gene from Synechocystis sp. PCC 6803 and characterization of a new mutation which confers resistance to the herbicide norflurazon. Plant Molecular Biology 18: 981-983.
15.Linden, H., Sandmann, G., Chamovitz, D., Hirschberg, J. und Böger, P. 1990. Biochemical characterization of Synechococcus mutants selected against the bleaching herbicide norflurazon. Pesticide Biochemistry and Physiology 36: 46-51.
16.Sandmann, G., Schneider, C. und Böger, P. 1996. A new non-radioactive assay of phytoene desaturase to evaluate bleaching herbicides. Verlag der Zeitschrift für Naturforschung. 51c:534-538.
17.Bartley, G.E., Viitanen, P.V., Pecker, I., Chamovitz, D., Hirschberg, J. und Scolnik, P. 1991. Molecular cloning and expression in photosynthetic bacteria of a soybean cDNA coding for phytoene desaturase, an enzyme of the carotenoid biosynthesis pathway. Proc. Natl. Acad. Sci. USA 88: 6532-6536.
18.Chamovitz, D., Pecker, I. und Hirschberg, J. 1991. The molecular basis of resistance to the herbicide norflurazon. Plant Molec. Biol. 16, 967-974.
19.Berthold P, Schmitt R, Mages W. (2002) "An engineered Streptomyces hygroscopicus aph 7" gene mediates dominant resistance against hygromycin B in Chlamydomonas reinhardtii. "Protist. 153(4):401-12.
20.Chamovitz D., Sandmann G., Hirschberg J. (1993) "Molecular and biochemical characterization of herbicide-resistant mutants of cyanobacteria reveals that phytoene desaturation is a rate-limiting step in Carotenoid Biosynthesis. J. Biol. Chem. 268: 17348-17353.
21.Boussiba, S., Fan, L. und Vonshak, A. (1992) Enhancement and determination of astaxanthin cumulation in green alga Haematococcus pluvialis. In Packer, L. (ed.) Methods in Enzymology. Academic Press, London, Vol. 213, pp. 371-386.
22.Cogoni, C. and G. Macino (1997). "Isolation of quelling-defective (qde) mutants impaired in posttranscriptional transgene-induced gene silencing in Neurospora crassa." Proc Natl Acad Sci U S A 94(19): 10233-8.
23.Davies, B.H. (1976) Carotenoids. In Goodwin, T.W. (ed.) Chemistry and Biochemistry of Plant Pigments. Academic Press, London, pp. 38-165.
24.Fabregas, J., A. Dominguez, et al. (2000). "Optimization of culture medium for the continuous cultivation of the microalga Haematococcus pluvialis." Appl Microbiol Biotechnol 53(5): 530-5.
25.Fraser, P. D., S. Romer, et al. (2002). "Evaluation of transgenic tomato plants expressing an additional phytoene synthase in a fruit-specific manner." Proc Natl Acad Sci.U S A_99(2): 1092-7.
26.Grunewald, K., M. Eckert, et al. (2000). "Phytoene desaturase is localized exclusively in the chloroplast and up-regulated at the mRNA level during accumulation of secondary carotenoids in Haematococcus pluvialis (Volvocales, chlorophyceae)." Plant Physiol 122(4): 1261-8.
27.Hallmann A, Sumper M. (1996) "The Chlorella hexose/H+ symporter is a useful selectable marker and biochemical reagent when expressed in Volvox." Proc Natl Acad Sci U S A. 93(2):669-73
28.Klein, T. M., E. D. Wolf, et al. (1987). "High-velocity microprojectiles for delivering nucleic acids into living cells." Nature(327): 70-73.
29.Kobayashi, M., T: Kakizono, et al. (1991). "Astaxanthin Production by a Green Alga, Haematococcus pluvialis Accompanied with Morphological Changes in Acetate Media." J.. Ferment. and Bioeng. 71(5): 335-339.
30.Mahmoud SS, Croteau RB. (2001) "Metabolic engineering of essential oil yield and composition in mint by altering expression of deoxyxylulose phosphate reductoisomerase and menthofuran synthase. "Proc Natl Acad Sci U S A. 98(15):15-20.
31.Maxwell, K. and G. N. Johnson (2000). "Chlorophyll fluorescence -a practical guide." J of Experim Bot 51(345): 659-668.
32.Misawa, N., S. Yamano, et al. (1993). "Functional expression of the Erwinia uredovora carotenoid biosynthesis gene crtl in transgenic plants showing an increase of beta-carotene biosynthesis activity and resistance to the bleaching herbicide norflurazon." Plant J 4(5): 833-40.
33.Misawa, N., K. Masamoto, et al. (1994). "Expression of Erwinia phytoene desaturase gene not only confers multiple resistance to herbicides interfering with carotenoid biosynthesis but also alters xanthophyll metabolism in transgenic plants." Plant J. 6(4): 481-489.
34.Niyogi, K. K., O. Bjorkman, et al. (1997). "Chlamydomonas Xanthophyll Cycle Mutants Identified by Video Imaging of Chlorophyll Fluorescence Quenching." Plant Cell 9(8): 1369-1380.
35.Pecker I, Chamovitz D, Linden H, Sandmann G, Hirschberg J. (1992) " A single polypeptide catalyzing the conversion of phytoene to zeta-carotene is transcriptionally regulated during tomato fruit ripening. " Proc Natl Acad Sci U S A 89(11):4962-6.
36.Steinbrenner, J. and H. Linden (2001). "Regulation of two carotenoid biosynthesis genes coding for phytoene synthase and carotenoid hydroxylase during stress-induced astaxanthin formation in the green alga Haematococcus pluvialis." Plant Physiol 125(2): 810-7.
37.Steinbrenner, J. and H. Linden (2003). "Light induction of carotenoid biosynthesis genes in the green alga Haematococcus pluvialis: regulation by photosynthetic redox control." Plant Mol Biol 52(2): 343-56.
38.Tanaka T, Morishita Y, Suzui M, Kojima T, Okumura A, Mori H (1994) "Chemoprevention of mouse urinary bladder carcinogenesis by the naturally occurring carotenoid astaxanthin." Carcinogenesis 15:15-19
39.Tanaka T, Kawamori T, Ohnishi M, Makita H, Mori H, Satoh K, Hara A. (1995) "Suppression of azoxymethane-induced rat colon carcinogenesis by dietary administration of naturally occurring xanthophylls astaxanthin and canthaxanthin during the postinitiation phase. Carcinogenesis. 16 (12):2957-63.

## Patentansprüche

1. Vektor umfassend
(a) eine DNA-Sequenz, die für das Protein Phytoen-Desaturase, welches an einer Position eine Resistenz gegenüber Herbiziden vermittelnde Aminosäuresubstitution besitzt, codiert, und
(b) eine Multiple Cloning Site (MCS), in die eine beliebige DNA-Sequenz einkloniert werden kann,
wobei die das Protein Phytoen-Desaturase kodierende DNA-Sequenz unter Beücksichtigung der Degeneration des genetischen Codes ein Protein gemäß SEQ ID NO:2 kodiert, wobei das Protein gemäß SEQ ID NO:2 einen Aminosäureaustausch von Leucin zu Arginin an Position 504 aufweist.

2. Vektor gemäß Anspruch 1, **dadurch gekennzeichnet dass**, der Vektor eine DNA-Sequenz (a) besitzt, die Transformanten eine Resistenz gegenüber Herbiziden vermittelt.

3. Vektor gemäß einem der Ansprüche 1 oder 2, **dadurch gekennzeichnet dass**, der Vektor eine DNA-Sequenz (a) besitzt, die Transformanten eine Resistenz gegenüber Bleichherbiziden, vermittelt.

4. Vektor nach Anspruch 3, **dadurch gekennzeichnet, dass** das Bleichherbizid Norflurazon ist.

5. Vektor gemäß einem der Ansprüche 1 bis 4, **dadurch gekennzeichnet dass** er die DNA-Sequenz SEQ ID NO:5 besitzt.

6. Vektor einem der Ansprüche 1 bis 5, **dadurch gekennzeichnet, dass** die in die MCS einzuklonierende beliebige DNA-Sequenz eine Codier-Sequenz ist.

7. Vektor gemäß Anspruch 6, **dadurch gekennzeichnet, dass** die Codier-Sequenz pflanzlicher Herkunft ist.

8. Vektor gemäß Anspruch 7, **dadurch gekennzeichnet, dass** die Codier-Sequenz wenigstens eine Promotor-Sequenz enthält.

9. Vektor gemäß Anspruch 8, **dadurch gekennzeichnet, dass** die PromotorSequenzen ausgewählt sind aus der Gruppe, die besteht aus *Haematococcus* Promotoren des Actingens und des Rubiscogens.

10. Vektor gemäß einem der Ansprüche 1 bis 9, **dadurch gekennzeichnet, dass** die Codier-Sequenz neben wenigstens einem Promotor-Gen ein zu exprimierendes Funktionsgen enthält.

11. Vektor gemäß Anspruch 10, **dadurch gekennzeichnet, dass** die Codier-Sequenz ausgewählt ist aus der Gruppe, die besteht aus Carotinoidbiosynthesegenen, Astaxanthinbiosynthesegenen und Isoprenoidbiosynthesegenen.

12. Vektor gemäß einem der Ansprüche 1 bis 11, **dadurch gekennzeichnet, dass** der Vektor aus einem frei erhältlichen Vektor hergestellt ist.

13. Verwendung eines Vektors gemäß einem der Anspruch 1 bis 12 zur Transformation von eukaryotischen Zellen.

14. Verwendung gemäß Anspruch 13 zur Transformation von einzelligen Pflanzenzellen.

15. Verwendung gemäß Anspruch 13 oder 14 zur Transformation von Algen-Zellen.

16. Verwendung gemäß Anspruch 15 zur Transformation von *H. pluvialis-*Zellen.

17. Verwendung des Vektors gemäß einem der Ansprüche 1 bis 12 als selektiver Marker zur Transformation.

18. Verwendung des Vektors gemäß Anspruch 17 als dominanter selektiver Marker zur Transformation.

19. Verfahren zur Transformation, **dadurch gekennzeichnet, dass** ein Vektor gemäß einem der Ansprüch 1 bis 12 verwendet wird.

20. Verfahren nach Anspruch 19, **dadurch gekennzeichnet, dass** die Transformation durch Partikelbeschuss durchgeführt wird.

21. Verfahren gemäß einem der Ansprüche 19 oder 20, **dadurch gekennzeichnet dass** der Partikelbeschuß mit 0,4 - 1,7 µm großen Wolfram-Partikeln bei einem Druck von 500 - 2500 psi und im Vakuum durchgeführt wird.

22. Transgene Pflanzenzelle und deren Nachkommen, **dadurch gekennzeichnet, dass** sie einen Vektor nach Anspruch 1 in ihren nukleären Genom enthält.

23. Transgene Pflanzenzelle und deren Nachkommen nach Anspruch 22, **dadurch gekennzeichnet dass** sie einen Einfach- oder Mehrfacheinbau der eingebrachten DNA ins nukleäre Genom aufweist.

24. Transgene Pflanzenzelle und deren Nachkommen nach einem der Ansprüche 22 und 23, **dadurch gekennzeichnet, dass** das eingebrachte Gen konsititutiv exprimiert wird.

## Claims

1. Vector comprising
(a) a DNA sequence which codes for the protein phytoene desaturase which has an amino acid substitution conferring resistance to herbicides at one position, and
(b) a multiple cloning site (MCS) into which any DNA sequence can be cloned,
wherein the DNA sequence coding for the protein phytoene desaturase codes for a protein according to SEQ ID NO:2 taking into consideration the degeneracy of the genetic code, where the protein according to SEQ ID NO:2 has an amino acid substitution of leucine by arginine at position 504.

2. Vector according to claim 1, **characterized in that** the vector has a DNA sequence (a) which confers a resistance to herbicides in the transformants.

3. Vector according to one of the claims 1 or 3, **characterized in that** the vector has a DNA sequence (a) which confers a resistance to bleaching herbicides in the transformants.

4. Vector according to claim 3, **characterized in that** the bleaching herbicide is norflurazon.

5. Vector according to one of the claims 1 to 4, **characterized in that** it has the DNA sequence SEQ ID NO:5.

6. Vector according to one of the claims 1 to 5, **characterized in that** the arbitrary DNA sequence to be cloned into the MCS is a coding sequence.

7. Vector according to claim 6, **characterized in that** the coding sequence is of plant origin.

8. Vector according to claim 7, **characterized in that** the coding sequence contains at least one promoter sequence.

9. Vector according to claim 8, **characterized in that** the promoter sequences are selected from the group consisting of *Haematococcus* promoters of the actin gene and of the rubisco gene.

10. Vector according to one of the claims 1 to 9, **characterized in that** the coding sequence contains a functional gene to be expressed in addition to at least one promoter gene.

11. Vector according to claim 10, **characterized in that** the coding sequence is selected from the group consisting of carotinoid biosynthesis genes, astaxanthine biosynthesis genes and isoprenoid biosynthesis genes.

12. Vector according to one of the claims 1 to 11, **characterized in that** the vector is prepared from a freely obtainable vector.

13. Use of a vector according to one of the claims 1 to 12 for the transformation of eukaryotic cells.

14. Use according to claim 13 for the transformation of single-celled plant cells.

15. Use according to claim 13 or 14 for the transformation of algal cells.

16. Use according to claim 15 for the transformation of *H. pluvialis* cells.

17. Use of a vector according to one of the claims 1 to 12 as a selective marker for transformation.

18. Use of the vector according to claim 17 as a dominant selective marker for transformation.

19. Method for transformation, **characterized in that** a vector according to one of the claims 1 to 12 is used.

20. Method according to claim 19, **characterized in that** the transformation is carried out by means of particle bombardment.

21. Method according to one of the claims 19 or 20, **characterized in that** the particle bombardment is carried out with tungsten particles of 0.4 - 1.7 µm in size at a pressure of 500 - 2500 psi and in a vacuum.

22. Transgenic plant cell and its progeny, **characterized in that** it contains a vector according to claim 1 in its nuclear genome.

23. Transgenic plant cell and its progeny according to claim 22, **characterized in that** it has a single or multiple incorporation of the introduced DNA into the nuclear genome.

24. Transgenic plant cell and its progeny according to one of the claims 22 and 23, **characterized in that** the introduced gene is expressed constitutively.

## Revendications

1. Vecteur comprenant
(a) une séquence d'ADN qui code la protéine phytoène-désaturase, qui possède à une position une substitution d'aminoacide conférant une résistance à l'égard des herbicides, et
(b) un site de clonage multiple (SCM) dans lequel une séquence d'ADN quelconque peut être clonée,
où la séquence d'ADN codant la protéine phytoène-désaturase code une protéine selon SEQ ID NO : 2 compte tenu de la dégénérescence du code génétique, où la protéine selon SEQ ID NO: 2 présente un échange d'aminoacides de leucine en arginine à la position 504.

2. Vecteur selon la revendication 1 **caractérisé en ce que** le vecteur possède une séquence d'ADN (a) qui confère aux transformants une résistance à l'égard des herbicides.

3. Vecteur selon l'une des revendications 1 et 2 **caractérisé en ce que** le vecteur possède une séquence d'ADN (a) qui confère aux transformants une résistance à l'égard des herbicides par chlorose.

4. Vecteur selon la revendication 3 **caractérisé en ce que** l'herbicide par chlorose est le norflurazon.

5. Vecteur selon l'une des revendications 1 à 4 **caractérisé en ce que** la séquence d'ADN possède SEQ ID NO : 5.

6. Vecteur selon l'une des revendications 1 à 5 **caractérisé en ce que** la séquence d'ADN quelconque à cloner dans le SCM est une séquence codante.

7. Vecteur selon la revendication 6 **caractérisé en ce que** la séquence codante est d'origine végétale.

8. Vecteur selon la revendication 7 **caractérisé en ce que** la séquence codante contient au moins une séquence promotrice.

9. Vecteur selon la revendication 8 **caractérisé en ce que** les séquences promotrices sont choisies dans le groupe qui consiste en les promoteurs de *Haematococcus* du gène de l'actine et du gène de la Rubisco.

10. Vecteur selon l'une des revendications 1 à 9 **caractérisé en ce que** la séquence codante contient un gène de fonction à exprimer outre au moins un gène de promoteur.

11. Vecteur selon la revendication 10 **caractérisé en ce que** la séquence codante est choisie dans le groupe qui consiste en les gènes de
biosynthèse de caroténoïdes, les gènes de biosynthèse d'astaxanthine et les gènes de biosynthèse d'isoprénoïdes.

12. Vecteur selon l'une des revendications 1 à 11 **caractérisé en ce que** le vecteur est produit à partir d'un vecteur librement accessible.

13. Utilisation d'un vecteur selon l'une des revendications 1 à 12 pour la transformation de cellules eucaryotes.

14. Utilisation selon la revendication 13 pour la transformation de cellules de plantes unicellulaires.

15. Utilisation selon la revendication 13 ou 14 pour la transformation de cellules d'algues.

16. Utilisation selon la revendication 15 pour la transformation de cellules de *H. pluvialis*.

17. Utilisation du vecteur selon l'une des revendications 1 à 12 comme marqueur sélectif pour la transformation.

18. Utilisation du vecteur selon la revendication 17 comme marqueur sélectif dominant pour la transformation.

19. Procédé de transformation **caractérisé en ce qu'**un vecteur selon l'une des revendications 1 à 12 est utilisé.

20. Procédé selon la revendication 19 **caractérisé en ce que** la transformation est réalisée par bombardement avec des particules.

21. Procédé selon l'une des revendications 19 et 20 **caractérisé en ce que** le bombardement avec des particules est réalisé avec des particules de tungstène d'une taille de 0,4 - 1,7 µm à une pression de 500 - 2500 psi et sous vide.

22. Cellule végétale transgénique et ses descendants **caractérisée en ce qu'**elle contient un vecteur selon la revendication 1 dans son génome nucléaire.

23. Cellule végétale transgénique et ses descendants selon la revendication 22 **caractérisée en ce qu'**elle présente dans le génome nucléaire une incorporation unique ou multiple de l'ADN introduit.

24. Cellule végétale transgénique et ses descendants selon l'une des revendications 22 et 23 **caractérisée en ce que** le gène introduit est exprimé de manière constitutive.


### SEQUENZPROTOKOLL

<110> Peter und Traudl Engelhorn-Stiftung zur Förderung der Biotechnologie
<120> Astaxanthinbiosynthese in Eukaryoten
<130> 34309P DE
<140> 10 2005 049 072.7
   <141> 2005-10-13
<160> 10
<170> PatentIn version 3.3
<210> 1
   <211> 6149
   <212> DNA
   <213> H. pluvialis
<220>
   <223> pds-Gen
<400> 1
   atcggtgagg ggttcaagtg ccagctcagt caaacatcgt gcattcagga ggcacagtta      60
   gaggcgtggt tgcagacgta ggtgagcagt tgggtgctgc tgctgctgtg atctgataca     120
   tgttgaaaac tgagcggaat ggccatgtat tgttgtttga ttactcatgg ccatgcactg     180
   agcatgcagc aagccatacc catccaggag ttgtcttcac ttcctttccc gcgcttgctt     240
   gctttcatcc gctttctttc catatgctgt gttgtgatcc ctccctcccc tcctccctcc     300
   ttaccctgcc ccacccctgg tggtgtgtgg ccaggtggct gtgacgcact gcctgctgag     360
   cgctgacctg gtgagcatga gcgccaggct gggccgagct gcctgggtgg ccaccactcg     420
   gctgtatgct gctcagcaag aggtggcagg ccaggcccat gtggcagcca tgcacctggt     480
   ggcaatcggg gatagggctg cggcagaagc agtgtacagg tgggcgcggg caggagcagg     540
   gacaggggct atagggtccc cggccaggtc taccctgact gctggggtta aggctagggc     600
   tagggctagg gctagggcta gggctagggc tagggctagg gctagggcta gggctagggc     660
   tagggctagg gctagggcta gggctagggc tagggctagg gctagggcta gggctaagga     720
   caaggggtgg gggcgaaggg tgggggtttg cagcagccag ggtcagccct gggccaacag     780
   tagctactgc acatccccac aatctcctac aggcagcaca ggctaacgtg cgcggactgc     840
   atcaccgggt acacagttga tgacatctta tcgaagcatc tcaccacacg acacatcaca     900
   attagttgca agggttcctg tcggtgttga ggtttgagtt ggtctgtatg caggggcctg     960
   atggaaaaac tgtagtagct gagtgagatt acccacagcc aagcatgttg ttccatgttg    1020
   aacaggtttt gtttggtctg atggaaaaac tgttgaaatg cttcctgtca tcgttccgct    1080
   gtacgccatt gtacgccgtt gactgatgga ctgattgttt gactgatgga agaactgtgt    1140
   agacctcagc cccctcagca cattagcata tctcgacaca tcagccatgc gcacaccgag    1200
   ttcgccttgt catgcgcagc caccctgtag aatgaagtca cgtaagtagt ggctgccgcg    1260
   aggtcgcctt ggtattgtct ctggcttgat atagcgctga ctgacgggtg gtgacggtgc    1320
   tcgtgcgcta tagatgccac tagaggcccg cccacctcgc gcctgagccg caaaacccgg 1380
   cggggtcgtc acgatgaggg catcttacct cgacgatgta ccgaccccgg gagctgtccc 1440
   gctggactgg cggatcgcgg ctgcccaact tgggcactga gcgttggctg tggatcaaca 1500
   tcattggcgc cgatgtcagc ctgggccagc aggtgcagcg gctgaccggc tatggaccgg 1560
   ctatggaccg gctacgatag cggatgcagc gttcctccgc atgcagagat gcactggacc 1620
   agtaccggag acggcgccag cgggatacac ggattagtgc gcctgaactg cggtgtgccc 1680
   catgaatgac catgccagcc ccaaggggcg gcattgaagc ggccctagag agctagtggc 1740
   aaggcagcac tagacaactt atgagctaag gtcagggagc tatgaggctg caagattagg 1800
   ggcctgattt gagttgttca gtcgcacgtc ttcacgacgt tacggctgcc atcttaccca 1860
   gtcggctaaa gcaggccacc accattggcg tcacgcgtga tcgtcttgaa atgacagcta 1920
   gtccaaacac caggctcagc tcgtatggtt tggcgagggc gtttcgtcgg ttgaacgacg 1980
   tgcctttaat gaaaattcgt ggccatacct tcatcgcttg agccaaccat ccagcatcaa 2040
   ctctgacaat gcagacagca atgcgtggcc aagccagcgg ttcaggatgc acttccagca 2100
   ggcaagctcg cggtcattgg tcaaggcgct ctgtgcgtga gcgaggtgct ctcagggtgg 2160
   ttgccaagga ctaccccacg ccggatttcc aatcatcgga cacataccag gaggccctgt 2220
   ccctgtcgac caagctgcga aatacacccc ggcccgccaa gcctttgcgc gttgtcattg 2280
   ctggggccgg cctggcaggc ctctcggctg caaaatacct ggcagatgca gggcaccacc 2340
   cagttgtgct ggagggccgc gacgtgctag gcggcaaggt aaggacttgc gagtgtcgaa 2400
   tgccacgccg gcccgttcca aacgcccaca agctgtccgg ccacctcaca aactcattgg 2460
   cgcacaggtg gccgcgtgga aggacgagga cggtgattgg tacgagactg gccttcacat 2520
   tttctttggt gcttacccca acatccagaa cctgttcaaa gagttgggca tccaagacag 2580
   gtaggaggtg cacccagtag tgtcaacgca ttgcctagca agcgcctcag tcaacaagac 2640
   caccatgagc actgttcttg atgtgggcat gtgcctgcct tgcaggctgc agtggaagga 2700
   gcattccatg atctttgcca tgccagacgc ccctggggag ttttctcgct ttgacttccc 2760
   tgagctgcct gcgccctgga acggcatcat cgccatcttg cggaacaacc agatgctcag 2820
   ctggcctgag aagatccggt ttgccatcgg cctgctgcct gctatcatct ttgggcagcg 2880
   ctactgtgaa gagcaggacg agctgaccgt gacagagtgg atgcgcaagc agggtgtgcc 2940
   agaccgagtc aatgaggaag tgttcatcgc catggccaag gccttgaact tcatcaaccc 3000
   tgacgaccta tccatgacag tggtgctcac agcactgaac cgcttcctgc aggcaagtgt 3060
   tgggcctagg cagtcagggg tcaggggcct gcagggccag cttgcatctg cccgaccttc 3120
   agggccgccg cctccctcac aaagcatgca ccctccacaa cccgcctgtc tgtctgccca 3180
   acaccagctt atggtccaaa tccaccacaa ccgcctctca ccctcctgcc cctgctttca 3240
   gcctgagcac tgcagtcctg tcaccagcaa atgtgtggcg gctgcacttc ttgtccctgc 3300
   cgccaatcgc tttcagtgtg gcctggccag cttgtgacag tggctagagc tccaagctct 3360
   gcaaagactg agacaagctg tggtttgcat tgcctgggct gcaggagcag catggcagca 3420
   agatggcttt cttggatggc gccccacctg agcgcctctg tcagccaatg gtggactact 3480
   tcaaggcacg aggcggcgac ctcatgttca actcccgggt caagcagatt gtgttgaatg 3540
   tgaggtccct ggctctcagc ctcgccgcct cctgcagacg ctgcatgcca tgtgcagtat 3600
   caccatgcca cacacgtcgg catcagctgc aggcctagct ctcgcgccgc agtcacagtt 3660
   gaacccaaca catgtgcaag tcatcaccct gtgcagcagg caggggcagg ctgggtgcct 3720
   gggcctgtca tccactgcag ccacagtctt gagtggaggg tacagtcatt aactctgccc 3780
   atgaggtgtc agagtctttc atgggtgcta tatggtcgct cttggcgtgc aggacgacaa 3840
   gagtgtcaag cacctggccc tcaccaatgg tcagacagtt gagggcgacc tctacatctc 3900
   agccatgcca ggtgtgtgcc tatggtgcag ctgggctgtg tgcccctaag cagccacaca 3960
   agcactggat gccatcaccc attgctgcat gacataggca caaggatgat agccaacgac 4020
   atggatgagg cacctgggca cagaggcaca cgttgtggcc tgcacagcct gagtcatgaa 4080
   ttgagggggg agagccaggt aggctgtaat tgtggcactg acctccacaa aaccataaca 4140
   caccagattg actgagtcca gttaggcttg ggggcttccc ttcagtttgt tcagaaccag 4200
   gatgcttggc tnctccctac catgcgctga catgtgtgct tgcattgatg tggtctcgca 4260
   gtggacatca tgaagatcct catgcctgac ccctgggcct ccatgcccta cttcaagcag 4320
   ctgaacggcc tggagggggt gcctgtgatc aacatccaca tctggttcga tcgcaagctg 4380
   actacggtgg accacctgtt gttcagtcgg tcaccgctgc tgtctgtgta cgctgacatg 4440
   agcaccacgt gcaaggagta tgctgacgac aagaagagca tgcttgagct ggtgtttgcg 4500
   ccggccaagg agtggattgg ccggcctgac gaggagatca tcgcagccac catgacagag 4560
   ctggagcggc tgttcccgac agaggtcagg gctgaccagt ccatggccaa ggtaggcggg 4620
   tcaggcacca gccagcactg ccaggcctgc aggttaagag gcttgtgagg ggtgcagctc 4680
   tgggcagata gcagctacag ccagctaacg gtgccggggt tgtgagtgaa tggcgcccac 4740
   tgcctgaagc accaccctgc tcagcttgct ctgctgtgcc agcattggct gcctgctgtc 4800
   gaagtacttg tggtgatggg tgatggtggc atgatgtgca gatcttgaag tacaaggtgg 4860
   tgaagactcc attgtctgtg tacaagtcca ctgctgggcg ggagaagttc aggtgagcct 4920
   cgcgacagcc gacagtggaa tgcactgcag cccttgtggc tgcctgactg gctttcttgt 4980
   cagccgctct ctcgtgacca taactgatgc tgccgtacct tatctgctgc tgctgctgtc 5040
   atgctggtgc taatggtggt gccccggtgc tgctgctgct gtgctgcaga cccactcagc 5100
   gctccccaat ctccaacttc taccttgcgg gtgattacac caagcagaag tacctgctcc 5160
   atggagggtg cagtgttctc cggcaagctg gtcaccgaag ccattgtaga ggactggagc 5220
   gctcggggtg tgaccagcag cgctgccagc cgccagcctg ccctggctgc agccggtgtg 5280
   gtggcagggt cggctgcagt gattggggcg gcgttggttg cagccagtgg tgcaatagcc 5340
   ggtgggatgt aagtgatgaa agaatggcac ctggtagatg aggcagcatt tatagaaatg 5400
   gcagcaccct cgctgcctaa tgcttgtggt gtatactatt gacctcggga gttgaggccc   5460
   gcagccattg tgtggctatc gcatggggtg gtgcatcgct gctcaatgcc gacaacattg   5520
   ggatggccta gaacttattg ctaggtagtg tgacctggac aagagattgg gtatgggtct   5580
   ggtgaggaag ggcaggcact atgggcgagc attctgagag gttgggcagg cgtgctgtag   5640
   tcctgcgatg agttggagtg tctaaacggc agtaacggat atggggtcac acggtttaca   5700
   cgaacattgc tgaaatcgag gatcatcacg aagcgtgcaa gaaacgtcat gcaacatgca   5760
   aacaagccaa aattcaaacg atggcgaacg tggcgagaaa ggaagggaag aaagcgaaag   5820
   gagcgggcgc tagggcgctg gcaagtgtag cggtcacgct gcgcgtaacc accacacccg   5880
   ccgcgcttaa tgcgccgcta cagggcgcgt cccattcgcc attcaggctg cgcaactgtt   5940
   gggaagggcg atcggtgcgg gcctcttcgc tattacgcca gctggcgaaa gggggatgtg   6000
   ctgcaaggcg attaagttgg gtaacgccag ggttttccca gtcacgacgt tgtaaaacga   6060
   cggccagtga attgtaatac gactcactat agggcgaatt gggtaccggg ccccccctcg   6120
   aggtcgacgg tatcgataag cttgatatc                                     6149
<210> 2
   <211> 570
   <212> PRT
   <213> H. pluvialis
<220>
   <223> Phytoen-Desaturase
<400> 2
   Met Gln Thr Thr Met Arg Gly Gln Ala Ser Gly Ser Gly Cys Thr Ser
   1               5                   10                  15
   Gly Arg Gln Ala Arg Gly His Trp Ser Arg Arg Ser Val Arg Glu Arg
               20                  25                  30
   Gly Ala Leu Arg Val Val Ala Lys Asp Tyr Pro Thr Pro Asp Phe Gln
           35                  40                  45
   Ser Ser Asp Thr Tyr Gln Glu Ala Leu Ser Leu Ser Thr Lys Leu Arg
       50                  55                  60
   Asn Ala Pro Arg Pro Ala Lys Pro Leu Arg Val Val Ile Ala Gly Ala
   65                  70                  75                  80
   Gly Leu Ala Gly Leu Ser Ala Ala Lys Tyr Leu Ala Asp Ala Gly His
                   85                  90                  95
   His Pro Val Val Leu Glu Gly Arg Asp Val Leu Gly Gly Lys Val Ala
               100                 105                 110
   Ala Trp Lys Asp Glu Asp Gly Asp Trp Tyr Glu Thr Gly Leu His Ile
           115                 120                 125
   Phe Phe Gly Ala Tyr Pro Asn Ile Gln Asn Leu Phe Lys Glu Leu Gly
   130 135 140
   Ile Gln Asp Arg Leu Gln Trp Lys Glu His Ser Met Ile Phe Ala Met
   145 150 155 160
   Pro Asp Ala Pro Gly Glu Phe Ser Arg Phe Asp Phe Pro Glu Leu Pro
   165 170 175
   Ala Pro Trp Asn Gly Ile Ile Ala Ile Leu Arg Asn Asn Gln Met Leu
   180 185 190
   Ser Trp Pro Glu Lys Ile Arg Phe Arg Ile Gly Leu Leu Pro Ala Ile
   195 200 205
   Ile Phe Gly Gln Arg Tyr Cys Glu Glu Gln Asp Glu Leu Thr Val Thr
   210 215 220
   Glu Trp Met Arg Lys Gln Gly Val Pro Asp Arg Val Asn Glu Glu Val
   225 230 235 240
   Phe Ile Ala Met Ala Lys Ala Leu Asn Phe Ile Asn Pro Asp Asp Leu
   245 250 255
   Ser Met Thr Val Val Leu Thr Ala Leu Asn Arg Phe Leu Gln Glu Gln
   260 265 270
   His Gly Ser Lys Met Ala Phe Leu Asp Gly Ala Pro Pro Glu Arg Leu
   275 280 285
   Cys Gln Pro Met Val Asp Tyr Phe Lys Ala Arg Gly Gly Asp Leu Met
   290 295 300
   Phe Asn Ser Arg Val Lys Gln Ile Val Leu Asn Asp Asp Lys Ser Val
   305 310 315 320
   Lys His Leu Ala Leu Thr Asn Gly Gln Thr Val Glu Gly Asp Leu Tyr
   325 330 335
   Ile Ser Ala Met Pro Val Asp Ile Met Lys Ile Leu Met Pro Asp Pro
   340 345 350
   Trp Ala Ser Met Pro Tyr Phe Lys Gln Leu Asn Gly Leu Glu Gly Val
   355 360 365
   Pro Val Ile Asn Ile His Ile Trp Phe Asp Arg Lys Leu Thr Thr Val
   370 375 380
   Asp His Leu Leu Phe Ser Arg Ser Pro Leu Leu Ser Val Tyr Ala Asp
   385 390 395 400
   Met Ser Thr Thr Ser Lys Glu Tyr Arg Asp Asp Lys Lys Ser Met Leu
   405 410 415
   Glu Leu Val Phe Ala Pro Ala Lys Glu Trp Ile Gly Arg Pro Asp Glu
   420 425 430
   Glu Ile Ile Ala Ala Thr Met Thr Glu Leu Glu Arg Leu Phe Pro Thr
   435 440 445
   Glu Val Arg Ala Asp Gln Ser Met Ala Lys Ile Leu Lys Tyr Lys Val
   450 455 460
   Val Lys Thr Pro Leu Ser Val Tyr Lys Ser Thr Ala Gly Arg Glu Lys
   465 470 475 480
   Phe Arg Pro Thr Gln Arg Ser Pro Ile Ser Asn Phe Tyr Leu Ala Gly
   485 490 495
   Asp Tyr Thr Lys Gln Lys Tyr Leu Ala Ser Met Glu Gly Ala Val Phe
   500 505 510
   Ser Gly Lys Leu Val Thr Glu Ala Ile Val Glu Asp Trp Ser Ala Arg
   515 520 525
   Gly Val Thr Ser Ser Ala Ala Ser Arg Gln Pro Ala Leu Ala Ala Ala
   530 535 540
   Gly Val Val Gly Arg Val Gly Ser Ser Asp Trp Ala Arg Trp Leu Gln
   545 550 555 560
   Pro Val Gly Ala Ile Ala Gly Gly Cys Lys
   565 570
<210> 3
   <211> 569
   <212> PRT
   <213> H. pluvialis
<220>
   <223> Proteinsequenz der Phytoen-Desaturase mit Leu504Arg Austausch
<400> 3
   Met Gln Thr Thr Met Arg Gly Gln Ala Ser Gly Ser Gly Cys Thr Ser
   1 5 10 15
   Gly Arg Gln Ala Arg Gly His Trp Ser Arg Arg Ser Val Arg Glu Arg
   20 25 30
   Gly Ala Leu Arg Val Val Ala Lys Asp Tyr Pro Thr Pro Asp Phe Gln
   35 40 45
   Ser Ser Asp Thr Tyr Gln Glu Ala Leu Ser Leu Ser Thr Lys Leu Arg
       50                  55                  60
   Asn Ala Pro Arg Pro Ala Lys Pro Leu Arg Val Val Ile Ala Gly Ala
   65                  70                  75                  80
   Gly Leu Ala Gly Leu Ser Ala Ala Lys Tyr Leu Ala Asp Ala Gly His
                   85                  90                  95
   His Pro Val Val Leu Glu Gly Arg Asp Val Leu Gly Gly Lys Val Ala
               100                 105                 110
   Ala Trp Lys Asp Glu Asp Gly Asp Trp Tyr Glu Thr Gly Leu His Ile
           115                 120                 125
   Phe Phe Gly Ala Tyr Pro Asn Ile Gln Asn Leu Phe Lys Glu Leu Gly
       130                 135                 140
   Ile Gln Asp Arg Leu Gln Trp Lys Glu His Ser Met Ile Phe Ala Met
   145                 150                 155                 160
   Pro Asp Ala Pro Gly Glu Phe Ser Arg Phe Asp Phe Pro Glu Leu Pro
                   165                 170                 175
   Ala Pro Trp Asn Gly Ile Ile Ala Ile Leu Arg Asn Asn Gln Met Leu
               180                 185                 190
   Ser Trp Pro Glu Lys Ile Arg Phe Arg Ile Gly Leu Leu Pro Ala Ile
           195                 200                 205
   Ile Phe Gly Gln Arg Tyr Cys Glu Glu Gln Asp Glu Leu Thr Val Thr
       210                 215                 220
   Glu Trp Met Arg Lys Gln Gly Val Pro Asp Arg Val Asn Glu Glu Val
   225                 230                 235                 240
   Phe Ile Ala Met Ala Lys Ala Leu Asn Phe Ile Asn Pro Asp Asp Leu
                   245                 250                 255
   Ser Met Thr Val Val Leu Thr Ala Leu Asn Arg Phe Leu Gln Glu Gln
               260                 265                 270
   His Gly Ser Lys Met Ala Phe Leu Asp Gly Ala Pro Pro Glu Arg Leu
           275                 280                 285
   Cys Gln Pro Met Val Asp Tyr Phe Lys Ala Arg Gly Gly Asp Leu Met
       290                 295                 300
   Phe Asn Ser Arg Val Lys Gln Ile Val Leu Asn Asp Asp Lys Ser Val
   305                 310                 315                 320
   Lys His Leu Ala Leu Thr Asn Gly Gln Thr Val Glu Gly Asp Leu Tyr
                   325                 330                 335
   Ile Ser Ala Met Pro Val Asp Ile Met Lys Ile Leu Met Pro Asp Pro
               340                 345                 350
   Trp Ala Ser Met Pro Tyr Phe Lys Gln Leu Asn Gly Leu Glu Gly Val
           355                 360                 365
   Pro Val Ile Asn Ile His Ile Trp Phe Asp Arg Lys Leu Thr Thr Val
       370                 375                 380
   Asp His Leu Leu Phe Ser Arg Ser Pro Leu Leu Ser Val Tyr Ala Asp
   385                 390                 395                 400
   Met Ser Thr Thr Ser Lys Glu Tyr Arg Asp Asp Lys Lys Ser Met Leu
                   405                 410                 415
   Glu Leu Val Phe Ala Pro Ala Lys Glu Trp Ile Gly Arg Pro Asp Glu
               420                 425                 430
   Glu Ile Ile Ala Ala Thr Met Thr Glu Leu Glu Arg Leu Phe Pro Thr
           435                 440                 445
   Glu Val Arg Ala Asp Gln Ser Met Ala Lys Ile Leu Lys Tyr Lys Val
       450                 455                 460
   Val Lys Thr Pro Leu Ser Val Tyr Lys Ser Thr Ala Gly Arg Glu Lys
   465                 470                 475                 480
   Phe Arg Pro Thr Gln Arg Ser Pro Ile Ser Asn Phe Tyr Leu Ala Gly
                   485                 490                 495
   Asp Tyr Thr Lys Gln Lys Tyr Arg Ala Ser Met Glu Gly Ala Val Phe
               500                 505                 510
   Ser Gly Lys Leu Val Thr Glu Ala Ile Val Glu Asp Trp Ser Ala Arg
           515                 520                 525
   Gly Val Thr Ser Ser Ala Ala Ser Arg Gln Pro Ala Leu Ala Ala Ala
       530                 535                 540
   Gly Val Val Gly Arg Val Gly Ser Ser Asp Trp Ala Arg Trp Leu Gln
   545                 550                 555                 560
   Pro Val Gly Ala Ile Ala Gly Gly Cys
                   565
<210> 4
   <211> 226
   <212> DNA
   <213> künstliche Sequenz
<220>
   <223> Nukleinsäuresequenz der MCS des erfindungsgemäßen Vektors Plat-pdsMOD4.1
<400> 4
   ttgtaaaacg acggccagtg aattgtaata cgactcacta tagggcgaat tgggtaccgg 60
   gccccccctc gaggtcgacg gtatcgataa gcttgatatc gaattcctgc agcccggggg 120
   atccactagt tctagagcgg ccgccaccgc ggtggagctc cagcttttgt tccctttagt 180
   gagggttaat ttcgagcttg gcgtaatcat ggtcatagct gtttcc 226
<210> 5
   <211> 8742
   <212> DNA
   <213> künstliche Sequenz
<220>
   <223> DNA-Vektor Plat-pdsMOD4.1
<400> 5
   cacctaaatt gtaagcgtta atattttgtt aaaattcgcg ttaaattttt gttaaatcag 60
   ctcatttttt aaccaatagg ccgaaatcgg caaaatccct tataaatcaa aagaatagac 120
   cgagataggg ttgagtgttg ttccagtttg gaacaagagt ccactattaa agaacgtgga 180
   ctccaacgtc aaagggcgaa aaaccgtcta tcagggcgat ggcccactac gtgaaccatc 240
   accctaatca agttttttgg ggtcgaggtg ccgtaaagca ctaaatcgga accctaaagg 300
   gagcccccga tttagagctt gacggggaaa gccatcggtg aggggttcaa gtgccagctc 360
   agtcaaacat cgtgcattca ggaggcacag ttagaggcgt ggttgcagac gtaggtgagc 420
   agttgggtgc tgctgctgct gtgatctgat acatgttgaa aactgagcgg aatggccatg 480
   tattgttgtt tgattactca tggccatgca ctgagcatgc agcaagccat acccatccag 540
   gagttgtctt cacttccttt cccgcgcttg cttgctttca tccgctttct ttccatatgc 600
   tgtgttgtga tccctccctc ccctcctccc tccttaccct gccccacccc tggtggtgtg 660
   tggccaggtg gctgtgacgc actgcctgct gagcgctgac ctggtgagca tgagcgccag 720
   gctgggccga gctgcctggg tggccaccac tcggctgtat gctgctcagc aagaggtggc 780
   aggccaggcc catgtggcag ccatgcacct ggtggcaatc ggggataggg ctgcggcaga 840
   agcagtgtac aggtgggcgc gggcaggagc agggacaggg gctatagggt ccccggccag 900
   gtctaccctg actgctgggg ttaaggctag ggctagggct agggctaggg ctagggctag 960
   ggctagggct agggctaggg ctagggctag ggctagggct agggctaggg ctagggctag 1020
   ggctagggct agggctaggg ctagggctaa ggacaagggg tgggggcgaa gggtgggggt 1080
   ttgcagcagc cagggtcagc cctgggccaa cagtagctac tgcacatccc cacaatctcc 1140
   tacaggcagc acaggctaac gtgcgcggac tgcatcaccg ggtacacagt tgatgacatc 1200
   ttatcgaagc atctcaccac acgacacatc acaattagtt gcaagggttc ctgtcggtgt 1260
   tgaggtttga gttggtctgt atgcaggggc ctgatggaaa aactgtagta gctgagtgag 1320
   attacccaca gccaagcatg ttgttccatg ttgaacaggt tttgtttggt ctgatggaaa 1380
   aactgttgaa atgcttcctg tcatcgttcc gctgtacgcc attgtacgcc gttgactgat 1440
   ggactgattg tttgactgat ggaagaactg tgtagacctc agccccctca gcacattagc 1500
   atatctcgac acatcagcca tgcgcacacc gagttcgcct tgtcatgcgc agccaccctg 1560
   tagaatgaag tcacgtaagt agtggctgcc gcgaggtcgc cttggtattg tctctggctt 1620
   gatatagcgc tgactgacgg gtggtgacgg tgctcgtgcg ctatagatgc cactagaggc 1680
   ccgcccacct cgcgcctgag ccgcaaaacc cggcggggtc gtcacgatga gggcatctta 1740
   cctcgacgat gtaccgaccc cgggagctgt cccgctggac tggcggatcg cggctgccca 1800
   acttgggcac tgagcgttgg ctgtggatca acatcattgg cgccgatgtc agcctgggcc 1860
   agcaggtgca gcggctgacc ggctatggac cggctatgga ccggctacga tagcggatgc 1920
   agcgttcctc cgcatgcaga gatgcactgg accagtaccg gagacggcgc cagcgggata 1980
   cacggattag tgcgcctgaa ctgcggtgtg ccccatgaat gaccatgcca gccccaaggg 2040
   gcggcattga agcggcccta gagagctagt ggcaaggcag cactagacaa cttatgagct 2100
   aaggtcaggg agctatgagg ctgcaagatt aggggcctga tttgagttgt tcagtcgcac 2160
   gtcttcacga cgttacggct gccatcttac ccagtcggct aaagcaggcc accaccattg 2220
   gcgtcacgcg tgatcgtctt gaaatgacag ctagtccaaa caccaggctc agctcgtatg 2280
   gtttggcgag ggcgtttcgt cggttgaacg acgtgccttt aatgaaaatt cgtggccata 2340
   ccttcatcgc ttgagccaac catccagcat caactctgac aatgcagaca gcaatgcgtg 2400
   gccaagccag cggttcagga tgcacttcca gcaggcaagc tcgcggtcat tggtcaaggc 2460
   gctctgtgcg tgagcgaggt gctctcaggg tggttgccaa ggactacccc acgccggatt 2520
   tccaatcatc ggacacatac caggaggccc tgtccctgtc gaccaagctg cgaaatacac 2580
   cccggcccgc caagcctttg cgcgttgtca ttgctggggc cggcctggca ggcctctcgg 2640
   ctgcaaaata cctggcagat gcagggcacc acccagttgt gctggagggc cgcgacgtgc 2700
   taggcggcaa ggtaaggact tgcgagtgtc gaatgccacg ccggcccgtt ccaaacgccc 2760
   acaagctgtc cggccacctc acaaactcat tggcgcacag gtggccgcgt ggaaggacga 2820
   ggacggtgat tggtacgaga ctggccttca cattttcttt ggtgcttacc ccaacatcca 2880
   gaacctgttc aaagagttgg gcatccaaga caggtaggag gtgcacccag tagtgtcaac 2940
   gcattgccta gcaagcgcct cagtcaacaa gaccaccatg agcactgttc ttgatgtggg 3000
   catgtgcctg ccttgcaggc tgcagtggaa ggagcattcc atgatctttg ccatgccaga 3060
   cgcccctggg gagttttctc gctttgactt ccctgagctg cctgcgccct ggaacggcat 3120
   catcgccatc ttgcggaaca accagatgct cagctggcct gagaagatcc ggtttgccat 3180
   cggcctgctg cctgctatca tctttgggca gcgctactgt gaagagcagg acgagctgac 3240
   cgtgacagag tggatgcgca agcagggtgt gccagaccga gtcaatgagg aagtgttcat 3300
   cgccatggcc aaggccttga acttcatcaa ccctgacgac ctatccatga cagtggtgct 3360
   cacagcactg aaccgcttcc tgcaggcaag tgttgggcct aggcagtcag gggtcagggg 3420
   cctgcagggc cagcttgcat ctgcccgacc ttcagggccg ccgcctccct cacaaagcat 3480
   gcaccctcca caacccgcct gtctgtctgc ccaacaccag cttatggtcc aaatccacca 3540
   caaccgcctc tcaccctcct gcccctgctt tcagcctgag cactgcagtc ctgtcaccag 3600
   caaatgtgtg gcggctgcac ttcttgtccc tgccgccaat cgctttcagt gtggcctggc 3660
   cagcttgtga cagtggctag agctccaagc tctgcaaaga ctgagacaag ctgtggtttg 3720
   cattgcctgg gctgcaggag cagcatggca gcaagatggc tttcttggat ggcgccccac 3780
   ctgagcgcct ctgtcagcca atggtggact acttcaaggc acgaggcggc gacctcatgt 3840
   tcaactcccg ggtcaagcag attgtgttga atgtgaggtc cctggctctc agcctcgccg 3900
   cctcctgcag acgctgcatg ccatgtgcag tatcaccatg ccacacacgt cggcatcagc 3960
   tgcaggccta gctctcgcgc cgcagtcaca gttgaaccca acacatgtgc aagtcatcac 4020
   cctgtgcagc aggcaggggc aggctgggtg cctgggcctg tcatccactg cagccacagt 4080
   cttgagtgga gggtacagtc attaactctg cccatgaggt gtcagagtct ttcatgggtg 4140
   ctatatggtc gctcttggcg tgcaggacga caagagtgtc aagcacctgg ccctcaccaa 4200
   tggtcagaca gttgagggcg acctctacat ctcagccatg ccaggtgtgt gcctatggtg 4260
   cagctgggct gtgtgcccct aagcagccac acaagcactg gatgccatca cccattgctg 4320
   catgacatag gcacaaggat gatagccaac gacatggatg aggcacctgg gcacagaggc 4380
   acacgttgtg gcctgcacag cctgagtcat gaattgaggg gggagagcca ggtaggctgt 4440
   aattgtggca ctgacctcca caaaaccata acacaccaga ttgactgagt ccagttaggc 4500
   ttgggggctt cccttcagtt tgttcagaac caggatgctt ggctnctccc taccatgcgc 4560
   tgacatgtgt gcttgcattg atgtggtctc gcagtggaca tcatgaagat cctcatgcct 4620
   gacccctggg cctccatgcc ctacttcaag cagctgaacg gcctggaggg ggtgcctgtg 4680
   atcaacatcc acatctggtt cgatcgcaag ctgactacgg tggaccacct gttgttcagt 4740
   cggtcaccgc tgctgtctgt gtacgctgac atgagcacca cgtgcaagga gtatgctgac 4800
   gacaagaaga gcatgcttga gctggtgttt gcgccggcca aggagtggat tggccggcct 4860
   gacgaggaga tcatcgcagc caccatgaca gagctggagc ggctgttccc gacagaggtc 4920
   agggctgacc agtccatggc caaggtaggc gggtcaggca ccagccagca ctgccaggcc 4980
   tgcaggttaa gaggcttgtg aggggtgcag ctctgggcag atagcagcta cagccagcta 5040
   acggtgccgg ggttgtgagt gaatggcgcc cactgcctga agcaccaccc tgctcagctt 5100
   gctctgctgt gccagcattg gctgcctgct gtcgaagtac ttgtggtgat gggtgatggt 5160
   ggcatgatgt gcagatcttg aagtacaagg tggtgaagac tccattgtct gtgtacaagt 5220
   ccactgctgg gcgggagaag ttcaggtgag cctcgcgaca gccgacagtg gaatgcactg 5280
   cagcccttgt ggctgcctga ctggctttct tgtcagccgc tctctcgtga ccataactga 5340
   tgctgccgta ccttatctgc tgctgctgct gtcatgctgg tgctaatggt ggtgccccgg 5400
   tgctgctgct gctgtgctgc agacccactc agcgctcccc aatctccaac ttctaccttg 5460
   cgggtgatta caccaagcag aagtaccgcg cctccatgga gggtgcagtg ttctccggca 5520
   agctggtcac cgaagccatt gtagaggact ggagcgctcg gggtgtgacc agcagcgctg 5580
   ccagccgcca gcctgccctg gctgcagccg gtgtggtggc agggtcggct gcagtgattg 5640
   gggcggcgtt ggttgcagcc agtggtgcaa tagccggtgg gatgtaagtg atgaaagaat 5700
   ggcacctggt agatgaggca gcatttatag aaatggcagc accctcgctg cctaatgctt 5760
   gtggtgtata ctattgacct cgggagttga ggcccgcagc cattgtgtgg ctatcgcatg 5820
   gggtggtgca tcgctgctca atgccgacaa cattgggatg gcctagaact tattgctagg 5880
   tagtgtgacc tggacaagag attgggtatg ggtctggtga ggaagggcag gcactatggg 5940
   cgagcattct gagaggttgg gcaggcgtgc tgtagtcctg cgatgagttg gagtgtctaa 6000
   acggcagtaa cggatatggg gtcacacggt ttacacgaac attgctgaaa tcgaggatca 6060
   tcacgaagcg tgcaagaaac gtcatgcaac atgcaaacaa gccaaaattc aaacgatggc 6120
   gaacgtggcg agaaaggaag ggaagaaagc gaaaggagcg ggcgctaggg cgctggcaag 6180
   tgtagcggtc acgctgcgcg taaccaccac acccgccgcg cttaatgcgc cgctacaggg 6240
   cgcgtcccat tcgccattca ggctgcgcaa ctgttgggaa gggcgatcgg tgcgggcctc 6300
   ttcgctatta cgccagctgg cgaaaggggg atgtgctgca aggcgattaa gttgggtaac 6360
   gccagggttt tcccagtcac gacgttgtaa aacgacggcc agtgaattgt aatacgactc 6420
   actatagggc gaattgggta ccgggccccc cctcgaggtc gacggtatcg ataagcttga 6480
   tatcgaattc ctgcagcccg ggggatccac tagttctaga gcggccgcca ccgcggtgga 6540
   gctccagctt ttgttccctt tagtgagggt taatttcgag cttggcgtaa tcatggtcat 6600
   agctgtttcc tgtgtgaaat tgttatccgc tcacaattcc acacaacata cgagccggaa 6660
   gcataaagtg taaagcctgg ggtgcctaat gagtgagcta actcacatta attgcgttgc 6720
   gctcactgcc cgctttccag tcgggaaacc tgtcgtgcca gctgcattaa tgaatcggcc 6780
   aacgcgcggg gagaggcggt ttgcgtattg ggcgctcttc cgcttcctcg ctcactgact 6840
   cgctgcgctc ggtcgttcgg ctgcggcgag cggtatcagc tcactcaaag gcggtaatac 6900
   ggttatccac agaatcaggg gataacgcag gaaagaacat gtgagcaaaa ggccagcaaa 6960
   aggccaggaa ccgtaaaaag gccgcgttgc tggcgttttt ccataggctc cgcccccctg 7020
   acgagcatca caaaaatcga cgctcaagtc agaggtggcg aaacccgaca ggactataaa 7080
   gataccaggc gtttccccct ggaagctccc tcgtgcgctc tcctgttccg accctgccgc 7140
   ttaccggata cctgtccgcc tttctccctt cgggaagcgt ggcgctttct catagctcac 7200
   gctgtaggta tctcagttcg gtgtaggtcg ttcgctccaa gctgggctgt gtgcacgaac 7260
   cccccgttca gcccgaccgc tgcgccttat ccggtaacta tcgtcttgag tccaacccgg 7320
   taagacacga cttatcgcca ctggcagcag ccactggtaa caggattagc agagcgaggt 7380
   atgtaggcgg tgctacagag ttcttgaagt ggtggcctaa ctacggctac actagaagga 7440
   cagtatttgg tatctgcgct ctgctgaagc cagttacctt cggaaaaaga gttggtagct 7500
   cttgatccgg caaacaaacc accgctggta gcggtggttt ttttgtttgc aagcagcaga 7560
   ttacgcgcag aaaaaaagga tctcaagaag atcctttgat cttttctacg gggtctgacg 7620
   ctcagtggaa cgaaaactca cgttaaggga ttttggtcat gagattatca aaaaggatct 7680
   tcacctagat ccttttaaat taaaaatgaa gttttaaatc aatctaaagt atatatgagt 7740
   aaacttggtc tgacagttac caatgcttaa tcagtgaggc acctatctca gcgatctgtc 7800
   tatttcgttc atccatagtt gcctgactcc ccgtcgtgta gataactacg atacgggagg 7860
   gcttaccatc tggccccagt gctgcaatga taccgcgaga cccacgctca ccggctccag 7920
   atttatcagc aataaaccag ccagccggaa gggccgagcg cagaagtggt cctgcaactt 7980
   tatccgcctc catccagtct attaattgtt gccgggaagc tagagtaagt agttcgccag 8040
   ttaatagttt gcgcaacgtt gttgccattg ctacaggcat cgtggtgtca cgctcgtcgt 8100
   ttggtatggc ttcattcagc tccggttccc aacgatcaag gcgagttaca tgatccccca 8160
   tgttgtgcaa aaaagcggtt agctccttcg gtcctccgat cgttgtcaga agtaagttgg 8220
   ccgcagtgtt atcactcatg gttatggcag cactgcataa ttctcttact gtcatgccat 8280
   ccgtaagatg cttttctgtg actggtgagt actcaaccaa gtcattctga gaatagtgta 8340
   tgcggcgacc gagttgctct tgcccggcgt caatacggga taataccgcg ccacatagca 8400
   gaactttaaa agtgctcatc attggaaaac gttcttcggg gcgaaaactc tcaaggatct 8460
   taccgctgtt gagatccagt tcgatgtaac ccactcgtgc acccaactga tcttcagcat 8520
   cttttacttt caccagcgtt tctgggtgag caaaaacagg aaggcaaaat gccgcaaaaa 8580
   agggaataag ggcgacacgg aaatgttgaa tactcatact cttccttttt caatattatt 8640
   gaagcattta tcagggttat tgtctcatga gcggatacat atttgaatgt atttagaaaa 8700
   ataaacaaat aggggttccg cgcacatttc cccgaaaagt gc 8742
<210> 6
   <211> 2187
   <212> DNA
   <213> künstliche Sequenz
<220>
   <223> Actin-Promotor SmaI-Fragment
<400> 6
   cccccgggca ggcagcggtg gggccagcag tggggcccaa tggcagcgcc tggggcctgg     60
   agggggtagc ttaggcgggg cgggcggggg gggcggggtg ggaggcggtg tgggcgccca    120
   ggcgttggct ctgctgcgtg ggggctgggc taggattggg tcaggccccc ctatgctgcc    180
   gccccccgcc ccaggccccg ctgggtctga gggccctgcc ccccgcgcat ctccctgggt    240
   gcggcagctg cagcggggca gccacgcccg cagccactcc tcgccagtgg acctgaccag    300
   cccgcagctc acccaggcgc tgctggacga gtggcatggg ggggaggagc cagggggggg    360
   ccctggtctg gcccctaggc acaacagctt tgacttgagg ctcagggccg gccaataatg    420
   gggtggttgg gaagggtagg ggggtccggg agggggktkg gkaagggtaa ggtagggtta    480
   ggtaaggtag gctaggctca gggtgttatg catggcaatg gtagcaggcg tagctgcagc    540
   tggggaagat tcatggtaaa cgtataaagg gacccagctg atggtgggcg aatgacggac    600
   acctgcatgt attctttaca tttcaagtat ttcaaagttg gtttgcaacg atgtggggct    660
   aggctatgca ataagactgc tacatgaggc cgggctgatc accgctcgtg acgtatggac    720
   gtatctgacg tgatcaacct gacccttacc gaaccctgac ctgcagtgcc caatccatga    780
   tatcagctgg tgctctgtgc cacactattg cagtttacag cgatggatgc ttacttggct    840
   tgcgttgctt ggattctgcg accgtggcct tccaatgcgg ttcacctttc aagccacgat    900
   caatatcaca agcatgggtt gcatgagcgc taaccacctg cctttggaga cttgcgcgcc    960
   atacccattg tgtgggatac agcacccctt ccacacaccc gagatcccca cggtcagctg   1020
   tcgcgcttaa cccgctcccc cctcgcgcct cttgctcgtg atcattgtgg gttgccgcga   1080
   cgtcaccgct ccgtcaacgt gctttcaaac agggatcaaa aatgctccat cacccggtgc   1140
   agagtctttg ctgcaagcag cacaatggct gaggagggag aggtatctgc ccttgtctgc   1200
   gacaatggct cggggatggt gaaagccggg ttcgctgggg atgatgcgcc ccgtgcagtc   1260
   ttccccagca ttgtgggcag gccgcgccac acaggcgtga tggtgggcat ggggcaaaag   1320
   gtaagaacgc tgtgtgcgac gaactgcatc gctgcagctg gcccagctca acgtggcgct   1380
   ctatgtgtgc aggactcata cgtcggcgat gaggcacagt ccaagcgagg catcctgacg   1440
   ctacgctacc caatcgagca cggcattgtc acaaactggg atgacatgga aaaaatttgg   1500
   catcacacat ggttcaacga gcttcgcggt aaaaaatttg gcatcacaca tggttcaacg   1560
   agcttcgcgt ggctccagag gtgagtagcg agtccctgag gtgcagcact ggctgccctg   1620
   tttgcctctg ctttagccac tgattccctg gtcagtccca ggccaggact gacatgcatc   1680
   tacatgcgtt gaagtgcaat gtcgcaagcc cagctcagcc acctatattg ggtgcagggc   1740
   gcgtgggttt gccttccttc ccctgtaatc tgtttccccg atgtacaaaa cgatagtgtg   1800
   gtaggcctac aaggaaatgg tgtcatgggg gtccactgtc acgcctgagc tgatgctggt   1860
   cacaaggctc gcagcacgcc aaagctcagc agggtggctc aggccatatg gctcaagtac   1920
   gctgtgcctc caagctgggg ccatgtcact gcatggcgcc tgcatgtgtt aggaaacaaa   1980
   cgctaggatg ggcgtgaggc ccctttcctg cccccttccc gcagctctag cagcagagcc   2040
   atgagttagg gctagggtta gtcatggggc tgccttgcta gcttcgaatg ccggctgagc   2100
   acattctagt tgcatgcatt ggatatgcac ttgcgtgcag cttcccgctc cccatccaga   2160
   catttgttgt ccagctcccc gcccggg                                         2187
<210> 7
   <211> 1300
   <212> DNA
   <213> künstliche Sequenz
<220>
   <223> rbsc-Promotor PstI-Fragment
<400> 7
   ctgcagggcc agccactaat gctgcaacgg gaccagtaaa aggaacaggt tcaggaccag     60
   caggtgtcga gtatcgcgcc agctttgtgt gtttccgtgc gtgtgtgttg gggcgctgtt    120
   acatgccatg ccatgccacg tcactgcgac gacttctgat tgcagcggca agagcccggg    180
   cagtcgctat ttgcagaaac gagaggtttt atctcttgag catgggggtg accattggtg    240
   aaacactcga ggacgatctc atgcattcaa tggatcttat tcgccataca tataaacacg    300
   cgacgaccga tattaaggaa tgcagccaca agcttcagta agtgatggag cacttgccgg    360
   atttgtttct tgcagacaag gcttcaacat tgtcgtaaaa taggagtagt aaaggtatgg    420
   acagtagcag gctgcgggcc gctgtgaacc ttatctcgaa agctgggata aagcattaaa    480
   gtccatcact ccaagagtgt aatcaccatg acaatctcta ttgggtcgcc aacaaggact    540
   gtcgggttca gacagtgaga caagcagcag caatggccac catcgcagcc cgctcaacca    600
   gctctgccgt ctcctgcggc cgcccttgca cgcagctgcg tgcaggtgcg cgctgccctg    660
   aagcccagcg tgaaggccgc tcccgcggtc tccaccgctg gcgccaacca gatgatggtc    720
   tggcagccca ttaacaacaa gtgcggaccc ttatccactt cctgaaaccg agctcgaccc    780
   cagcctgcga cccgtcgtaa cacggcattc actgcctctt cctcgcaggc aatacgagac    840
   tttctcttac ttgcctccct tgacatccga ccagattgct cgccaggtcg actatgtcgt    900
   tggcaacggt tggattccat gcctggagtt cgctgacgcc agccaggcct atgtcagcaa    960
   cgccagcaca gtgcgctttg gctccgtgtc cgctgtgagt gtcttaggag acatgcgtac   1020
   cctatgcctt tctcgcccgt cgcctgctgc tcatgattcc cagtcgattg ctctccggac   1080
   tgacttttat cgtgtttgac ttttgcccga tcgcagtgct actacgacaa caggtactgg   1140
   acactgtgga agctgcccat gttcggttgc actgacccct ccgccgtgct gactgagatc   1200
   agccgcgcct ccaaggcctt cccccaggcc tacattcgca tggtgtgtag gaacagtgtt   1260
   cgcagcagca ttgtgtaaag cagcgtccgc tgttctgcag                         1300
<210> 8
   <211> 31
   <212> DNA
   <213> künstliche Sequenz
<220>
   <223> oligonukleotid
<400> 8
   ccaagcagaa gtaccgcgcc tccatggagg g 31
<210> 9
   <211> 31
   <212> DNA
   <213> künstliche Sequenz
<220>
   <223> oligonukleotid
<400> 9
   ccctccatgg aggcgcggta cttctgcttg g 31
<210> 10
   <211> 474
   <212> PRT
   <213> synechococcusPCC7942
<220>
   <223> Phytoen-Desaturase
<400> 10
   Met Arg Val Ala Ile Ala Gly Ala Gly Leu Ala Gly Leu Ser Cys Ala
   1               5                   10                  15
   Lys Tyr Leu Ala Asp Ala Gly His Thr Pro Ile Val Tyr Glu Arg Arg
               20                  25                  30
   Asp Val Leu Gly Gly Lys Val Ala Ala Trp Lys Asp Glu Asp Gly Asp
           35                  40                  45
   Trp Tyr Glu Thr Gly Leu His Ile Phe Phe Gly Ala Tyr Pro Asn Met
       50                  55                  60
   Leu Gln Leu Phe Lys Glu Leu Asn Ile Glu Asp Arg Leu Gln Trp Lys
   65                  70                  75                  80
   Ser His Ser Met Ile Phe Asn Gln Pro Thr Lys Pro Gly Thr Tyr Ser
                   85                  90                  95
   Arg Phe Asp Phe Pro Asp Ile Pro Ala Pro Ile Asn Gly Val Ala Ala
               100                 105                 110
   Ile Leu Ser Asn Asn Asp Met Leu Thr Trp Glu Glu Lys Ile Lys Phe
           115                 120                 125
   Gly Leu Gly Leu Leu Pro Ala Met Ile Arg Gly Gln Ser Tyr Val Glu
       130                 135                 140
   Glu Met Asp Gln Tyr Ser Trp Thr Glu Trp Leu Arg Lys Gln Asn Ile
   145                 150                 155                 160
   Pro Glu Arg Val Asn Asp Glu Val Phe Ile Ala Met Ala Lys Ala Leu
                   165                 170                 175
   Asn Phe Ile Asp Pro Asp Glu Ile Ser Ala Thr Val Val Leu Thr Ala
               180                 185                 190
   Leu Asn Arg Phe Leu Gln Glu Lys Lys Gly Ser Met Met Ala Phe Leu
           195                 200                 205
   Asp Gly Ala Pro Pro Glu Arg Leu Cys Gln Pro Ile Val Glu His Val
   210 215 220
   Gln Ala Arg Gly Gly Asp Val Leu Leu Asn Ala Pro Leu Lys Glu Phe
   225 230 235 240
   Val Leu Asn Asp Asp Ser Ser Val Gln Ala Phe Arg Ile Ala Gly Ile
   245 250 255
   Lys Gly Gln Glu Glu Gln Leu Ile Glu Ala Asp Ala Tyr Val Ser Ala
   260 265 270
   Leu Pro Val Asp Pro Leu Lys Leu Leu Leu Pro Asp Ala Trp Lys Ala
   275 280 285
   Met Pro Tyr Phe Gln Gln Leu Asp Gly Leu Gln Gly Val Pro Val Ile
   290 295 300
   Asn Ile His Leu Trp Phe Asp Arg Lys Leu Thr Asp Ile Asp His Leu
   305 310 315 320
   Leu Phe Ser Arg Ser Pro Leu Leu Ser Val Tyr Ala Asp Met Ser Asn
   325 330 335
   Thr Cys Arg Glu Tyr Glu Asp Pro Asp Arg Ser Met Leu Glu Leu Val
   340 345 350
   Phe Ala Pro Ala Lys Asp Trp Ile Gly Arg Ser Asp Glu Asp Ile Leu
   355 360 365
   Ala Ala Thr Met Ala Glu Ile Glu Lys Leu Phe Pro Gln His Phe Ser
   370 375 380
   Gly Glu Asn Pro Ala Arg Leu Arg Lys Tyr Lys Ile Val Lys Thr Pro
   385 390 395 400
   Leu Ser Val Tyr Lys Ala Thr Pro Gly Arg Gln Gln Tyr Arg Pro Asp
   405 410 415
   Gln Ala Ser Pro Ile Ala Asn Phe Phe Leu Thr Gly Asp Tyr Thr Met
   420 425 430
   Gln Arg Tyr Leu Ala Ser Met Glu Gly Ala Val Leu Ser Gly Lys Leu
   435 440 445
   Thr Ala Gln Ala Ile Ile Ala Arg Gln Asp Glu Leu Gln Arg Arg Ser
   450 455 460
   Ser Gly Arg Pro Leu Ala Ala Ser Gln Ala
   465 470